(19) **Europäisches Patentamt / European Patent Office / Office européen des brevets**

(11) **EP 4 729 171 A1**

(12) **EUROPEAN PATENT APPLICATION**

(43) Date of publication:
22.04.2026 Bulletin 2026/17

(21) Application number: 25209059.2

(22) Date of filing: 16.10.2025

(51) International Patent Classification (IPC):
*B01J 23/80* [(2006.01)]  *B01J 37/02* [(2006.01)]
*B01J 37/03* [(2006.01)]  *C07C 29/156* [(2006.01)]
*C07C 31/08* [(2006.01)]  *B01J 29/46* [(2006.01)]
*B01J 35/64* [(2024.01)]

(52) Cooperative Patent Classification (CPC):
(C-Sets available)
**B01J 37/0236; B01J 23/002; B01J 23/80;
B01J 29/46; B01J 35/45; B01J 35/613;
B01J 35/633; B01J 35/643; B01J 35/647;
B01J 37/0201; B01J 37/031; B01J 37/18;
C07C 29/156;** B01J 2235/30; B01J 2523/00

(Cont.)

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **18.10.2024 EP 24207402**

(71) Applicant: **Kemijski Institut
1000 Ljubljana (SI)**

(72) Inventors:
• KOSTYNIUK, Andrii
**1000 Ljubljana (SI)**
• LIKOZAR, Bla
**1000 Ljubljana (SI)**

(74) Representative: **Weickmann & Weickmann
PartmbB
Postfach 860 820
81635 München (DE)**

(54) **CO2 HYDROGENATION TO HIGH-CONCENTRATED ETHANOL OVER RUBIDIUM-MODIFIED TRIMETALLIC CATALYST**

(57) The present invention relates to the direct hydrogenation of $CO_2$ into ethanol and higher alcohols using optimized xRb/CuZnFe catalysts. This document showcases not only their enhanced performance and high selectivity but also offers valuable insights into catalyst design and the underlying reaction mechanisms. In addition, various factors such as Rb loading, reaction temperature, and time-on-stream were investigated.

**Fig. 8**

(a)

(Cont. next page)

EP 4 729 171 A1

(52) Cooperative Patent Classification (CPC): (Cont.)

C-Sets
**C07C 29/156, C07C 31/08;**
B01J 2523/00, B01J 2523/14, B01J 2523/17,
B01J 2523/27, B01J 2523/842

**Description**

Every day, large amounts of greenhouse gases (GHGs) are emitted into the atmosphere, contributing to global warming, with $CO_2$ making up 72% of total GHG emissions. This dominance is mainly due to the extensive burning of fossil fuels, and $CO_2$ emissions have been steadily increasing, reaching 33.9 billion metric tons globally in 2018. To combat this, carbon capture and utilization (CCU) and carbon capture and storage (CCS) are crucial strategies, with CCU being seen as a particularly promising solution for reducing $CO_2$ emissions and addressing climate change. Utilizing $CO_2$ as a resource for chemical processes, alongside renewable hydrogen production from water electrolysis, provides an environmentally sustainable approach to reducing $CO_2$ emissions while generating valuable products like CO, $CH_4$, light olefins, aromatics, dimethyl ether, formates, and alcohols. Among the various methods, one of the most promising is the direct thermocatalytic hydrogenation of $CO_2$ into ethanol using multifunctional heterogeneous catalysts. Ethanol can be synthesized through methods like ethylene hydration using a solid acid catalyst or fermentation of sugar, grain crops, and waste biomass. While fermentation is more sustainable and environmentally friendly than ethylene hydration, it faces limitations due to ethanol's toxicity to yeast, requiring distillation for higher concentrations, and raises ethical concerns about food versus fuel when using crops like corn and sugar cane.

The pursuit of methods for converting $CO_2$ into ethanol through hydrogenation is a significant area of interest, albeit one fraught with challenges in its development. Traditionally, noble-metal-based catalysts (such as Pd, Pt, and Rh) have been employed to overcome the initial hurdles involving C-O activation and subsequent C-C coupling in the hydrogenation of $CO_2$ to ethanol. However, the steep cost associated with these catalysts has prompted a shift from conventional approaches to the exploration of more abundant transition metals. Cu-based catalysts demonstrate high activity in the synthesis of methanol from $CO_2$ or CO, yet they display reduced selectivity towards $C_{2+}$ oxygenates; concurrently, alkali metals play crucial roles as promoters in Cu-catalysed ethanol synthesis from syngas, with selectivity ranking as Cs > K > Na > Li.

Recently, in a breakthrough study, Xu et al. introduced a novel Cs-promoted Cu-Fe-Zn catalyst tailored for the precise synthesis of $C_{2+}$ alcohols. This catalyst demonstrated $C_{2+}$ alcohols STY of 1.47 mmol $g_{cat}^{-1}$ $h^{-1}$. This remarkable achievement is rooted in the intricate synergy between the Cs promoter, Cu-ZnO, and copper-iron carbide, forming interfaces within the material structure. The pivotal role of Cs is evident as it orchestrates the CO insertion reaction by fine-tuning the hydrogenation capabilities of the CuFeZn catalysts. However, despite this significant advancement, it is worth noting that the achieved STY is not yet suitable for large-scale applications. Yang and co-authors subsequently prepared a CuFeZn catalyst using 2,6-pyridine dicarboxylic acid (PDA), achieving remarkable results, including the highest $C_{2+}OH$/-ROH fraction of 95.3% and a $C_{2+}$ alcohols STY of 58.2 mg/mL$_{cat}$/h, all while maintaining a $CO_2$ conversion rate of 10.6%. The authors attributed this exceptional catalytic performance to the coordination between PDA and the active metal ions, which facilitates electron transfer and the reduction of metallic oxides. Consequently, this coordination results in the even distribution of active components. Regrettably, it should be noted that despite its impressive performance, this catalyst exhibits a lower STY compared to the previous one utilizing Cs. Achieving efficient $CO_2$ conversion to ethanol remains challenging due to low selectivity, productivity, and insufficient catalyst stability. The direct conversion of $CO_2$ to ethanol, more complex than multi-step reactions, continues to deliver poor yields and selectivity, driving the search for innovative strategies in CO2 hydrogenation for ethanol production.

**Summary of the invention**

The present invention relates to the direct hydrogenation of $CO_2$ into ethanol and higher alcohols using optimized xRb/CuZnFe catalysts. This document showcases not only their enhanced performance and high selectivity but also offers valuable insights into catalyst design and the underlying reaction mechanisms. In addition, various factors such as Rb loading, reaction temperature, and time-on-stream were investigated. The described catalysts have ranked among the top in terms of STY of ethanol in the literature. The xRb/CuZnFe catalysts were extensively characterized using several advanced techniques, including BET (Brunauer-Emmett-Teller) surface area analysis, XRD (X-ray diffraction), XPS (X-ray photoelectron spectroscopy), SEM (Scanning electron microscopy), STEM (Scanning transmission electron microscopy), EDX (Energy-dispersive X-ray spectroscopy), and ICP-OES (inductively coupled plasma optical emission spectroscopy). BET analysis revealed the surface area and pore distribution crucial for active site exposure. XRD identified the crystalline phases present in the catalyst, while XPS provided insights into the chemical states of the active elements, which are essential for catalytic performance. SEM, STEM, and EDX mapping offered detailed morphological insights and elemental distribution, highlighting the homogeneity and synergies among the catalyst components. Additionally, ICP-OES was employed to quantify the precise elemental composition of the catalyst, ensuring accurate assessment of Rb loading and its impact on catalytic behaviour. These analyses collectively elucidated the underlying reaction mechanisms, guiding the optimization of the catalyst for enhanced $CO_2$ hydrogenation to ethanol.

**Detailed description**

The present invention provides an improved hydrogenation catalyst comprising or essentially consisting of Cu-Zn-Fe mixed oxide particles loaded with Rb in an amount of 2-8 wt.%, based on the total weight of the loaded Cu-Zn-Fe mixed oxide particles. These catalyst particles demonstrate effective and selective synthesis of $C_{2+}$ alcohols through $CO_2$ hydrogenation with corresponding conversion.

Preferably, Rb is loaded in an amount of 3-6 mol %, more preferably 3.5-5 mol, based on the total weight of the loaded Cu-Zn-Fe mixed oxide particles. Especially preferred is a loading with about 4 mol % of Rb. 4% doping level induces an optimal electronic environment that may enhance the catalytic properties of the CuZnFe catalyst. Rb-loading can be determined using SEM-EDX analysis. Optionally, the particles are additionally loaded with Cs.

Cu-Zn-Fe mixed oxide particles are essentially composed of Cu, Zn, and Fe oxides. Their respective amounts may differ. In preferred aspects, the molar ratio of Cu : Zn : Fe in the mixed oxide particles is in a range of 1 : 1-2 : 2-3, preferably about 1 : 1 : 2.

Based on the total weight of the Rb- or Cs-loaded Cu-Zn-Fe mixed oxide particles, Cu may be comprised in an amount of 10-25 wt.%, preferably 18-22 wt.%, more preferably about 20 wt.%. Zn may be comprised in an amount of 10-25 wt.%, preferably 12-16 wt.%, more preferably about 15 wt.%, and Fe may be comprised in an amount of 33-45 wt.%, preferably 35-38 wt.%, more preferably about 37 wt.%. The introduction of Fe at optimized level plays a critical role in electronic balance and structural stabilization (as shown by XPS and in situ DRIFTS).

The catalyst of the invention is preferably a powder comprising or essentially consisting of particles with an average particle size in a range of 10-50 nm, more preferably 15-30 nm as determined by scanning transmission electron microscopy in bright-field mode (STEM-BF).

The Cu-Zn-Fe mixed oxide particles loaded with Rb are porous particles. In preferred aspects, they comprise mesopores and micropores. Mesopores are pores with diameters between 2 and 50 nm. Micropores are pores spaller than 2 nm in diameter. In preferred embodiments, the average pore diameter is in a range of 5-20 nm, more preferably 6-18 nm, as determined from the desorption branch of nitrogen adsorption isotherms using the Barrett-Joyner-Halenda (BJH) method. This porosity yields an exceptional catalytic activity of the Rb loaded particles. The incorporation of Rb leads to the formation of a more open and porous structure. The increased porosity is beneficial as it can enhance the accessibility of reactants to the active sites of the catalyst, potentially improving catalytic performance. Additionally, the surface of the particles appears smoother compared to the CuZnFe catalyst, which might influence the interaction with reactants.

According to another embodiment, the invention provides a method for preparing the hydrogenation catalyst described herein. Generally, the catalyst can be prepared in a two-step process or in a one-step process. "Two-step" means that Cu-Zn-Fe mixed oxide particles are prepared and subsequently loaded with Rb, whereas a "one-step" process directly yields Rb-loaded particles.

Thus, a first method for preparing the hydrogenation catalyst comprises the steps of:

(i) providing Cu-Zn-Fe mixed oxide particles;
(ii) impregnating the Cu-Zn-Fe mixed oxide particles with a solution of a Rb salt, preferably an aqueous solution of $CsNO_3$ or $RbNO_3$;
(iii) drying the impregnated particles, preferably by heating to a temperature in a range of 50-150°C, more preferably 60-130°C, in particular about 110°C;
(iv) calcination of the dried particles, preferably at a temperature in a range of 300-600°C, more preferably 350-500°C, in particular about 400°C.

Providing Cu-Zn-Fe mixed oxide particles in step (i) may comprise the following steps:

(i.1) coprecipitating Cu, Zn and Fe salts from a mixed solution comprising salts of Cu, Zn and Fe, in particular in a molar ratio Cu:Zn:Fe of 1:1-2:2-3, preferably about 1:1:2;
(i.2) drying the precipitate, preferably by heating to a temperature in a range of 50-150°C, more preferably 60-130°C, in particular about 110°C and
(i.3) calcination of the dried precipitate, preferably at a temperature in a range of 300-600°C, more preferably 350-500°C, in particular about 400°C.

In step (i.1), the mixed (aqueous) solution is preferably prepared by first adding the Fe salt, followed by the Cu salt, and then the Zn salt, in the respective molar ratios. Preferably, the respective salts are added as an aqueous solution thereof.

In step (ii) of impregnating the Cu-Zn-Fe mixed oxide particles, they are contacted with a solution of a Rb salt, preferably an aqueous solution thereof. Suitable salts for use in the method of the invention include Rb oxides, $RbNO_3$, $Rb_2SO_4$, RbF, RbCl, RbBr, Rbl, $Rb_3PO4$, $Rb_2CO_3$, $Rb_2C_2O_4$, $CH_3CO_2Rb$ (with varying Rb loading, preferably from 1 to 10), or combinations thereof. Nitrates of Rb are particularly preferred.

Step (ii) may further comprise adding one or more additional alkali elements, such as Li, Na, K or combinations thereof, preferably as an aqueous solution comprising a Li, Na, or K salt or a combination thereof. Suitable salts of these alkali elements include Li oxides, $LiNO_3$, $Li_2SO_4$, LiF, LiCl, LiBr, LiI, $Li_3PO_4$, $Li_2CO_3$, $Li_2C_2O_4$, $CH_3CO_2Li$ (with varying Li loading, preferably from 1 to 10), Na oxides, $NaNO_3$, $Na_2SO_4$, NaF, NaCl, NaBr, NaI, $Na_3PO_4$, $Na_2CO_3$, $Na_2C_2O_4$, $CH_3CO_2Na$ (with varying Na loading, preferably from 1 to 10), K oxides, $KNO_3$, $K_2SO_4$, KF, KCl, KBr, KI, $K_3PO_4$, $K_2CO_3$, $K_2C_2O_4$, $CH_3CO_2K$ (with varying K loading, preferably from 1 to 10), or combinations thereof.

A second method for preparing the hydrogenation catalyst in a one-step process comprises the steps of:

(i) providing a mixed solution comprising salts of Cu, Fe, Zn, and Rb, preferably nitrates, in particular in a molar ratio Cu:Zn:Fe of 1:1-2:2-3, preferably about 1:1:2;

(ii) inducing coprecipitation of Rb-doped CuZnFe precipitate;

(iii) drying the precipitate, preferably by heating to a temperature in a range of 50-150°C, more preferably 60-130°C, in particular about 110°C; and

(iv) calcination of the dried precipitate, preferably at a temperature in a range of 300-600°C, more preferably 350-500°C, in particular about 400°C.

In this one-step process, salts of Rb can be selected from Rb oxides, $RbNO_3$, $Rb_2SO_4$, RbF, RbCl, RbBr, RbI, $Rb_3PO4$, $Rb_2CO_3$, $Rb_2C_2O_4$, $CH_3CO_2Rb$ (with varying Rb loading, preferably from 1 to 10), or combinations thereof. Nitrates of Rb are particularly preferred.

The mixed solution in step (i) may further comprise salts of one or more additional alkali elements, in particular salts of Li, Na, or K, or combinations thereof. Suitable salts of these alkali elements include Li oxides, $LiNO_3$, $Li_2SO_4$, LiF, LiCl, LiBr, LiI, $Li_3PO_4$, $Li_2CO_3$, $Li_2C_2O_4$, $CH_3CO_2Li$ (with varying Li loading, preferably from 1 to 10), Na oxides, $NaNO_3$, $Na_2SO_4$, NaF, NaCl, NaBr, NaI, $Na_3PO_4$, $Na_2CO_3$, $Na_2C_2O_4$, $CH_3CO_2Na$ (with varying Na loading, preferably from 1 to 10), K oxides, $KNO_3$, $K_2SO_4$, KF, KCl, KBr, KI, $K_3PO_4$, $K_2CO_3$, $K_2C_2O_4$, $CH_3CO_2K$ (with varying K loading, preferably from 1 to 10), or combinations thereof.

Another aspect of the present invention relates to a method for preparing ethanol by hydrogenation of $CO_2$, comprising

(i) reacting $H_2$ and $CO_2$ in a reactor the presence of a catalyst described hereinabove; and

(ii) isolating ethanol from a reaction product obtained in (i).

The reaction can be carried out in a packed bed continuous flow reactor comprising the catalyst of the invention. $H_2$ and $CO_2$ are supplied to the reactor with a gas feed rate GHSV in a range of preferably 9000-11000 $h^{-1}$, more preferably 9500-10500 $h^{-1}$, in particular about 10000 $h^{-1}$. Accordingly, the reactor feed rate can be in a range of 0.01-0.5 g/min, preferably 0.025-0.035 g/min, more preferably about 0.03 g/min or 20-100 ml/min, preferably 50-70 ml/min, more preferably about 60 ml/min.

The reaction can be carried out at a temperature in a range of 200-400°C. Preferably, the temperature is increased during the reaction time, for example with a first period at a temperature in a range of 180-220°C, preferably for 6-10 h, increasing the temperature to a higher temperature in a range of 220-270°C and holding the temperature for a second period, preferably for 1-5 h, increasing the temperature to a higher temperature in a range of 270-350°C and holding the temperature for a third period, preferably for 3-8 h.

The pressure in the reactor can controlled to be in a range of 15-25 bar, preferably 18-22 bar, more preferably about 20 bar.

The invention will be further described by the following Figures and Examples, which are not intended to limit the scope of the invention defined by the appended claims.

**Figures**

Fig. 1    $N_2$ adsorption-desorption isotherms (a) and BJH pore size distribution (b) of the CuZnFe and xRb/CuZnFe catalysts.

Fig. 2    XRD patterns of the CuZnFe and xRb/CuZnFe catalysts.

Fig. 3    SEM scans of CuZnFe - (a, c) and 4%Rb/CuZnFe samples - (b, d).

**Fig. 4**        STEM-BF images of CuZnFe - (a) and 4%Rb/CuZnFe - (b) samples.

**Fig. 5**        STEM images and EDX elemental mappings of (a) CuZnFe and (b) 4%Rb/CuZnFe

**Fig. 6**        XPS spectra of (a) - survey, (b) - C 1s, (c) - O 1s, (d) - Cu 2p, (e) -Zn 2p, (f) - Fe 2p, (g) - Rb 3d in the CuZnFe and xRb/CuZnFe catalysts.

**Fig. 7**        $CO_2$ conversion - (a), STY - (b), selectivity of $C_{2+}$ alcohols and/or EtOH - (c) and MeOH selectivity - (d) over CuZnFe and xRb/CuZnFe catalysts in the gas phase. Reaction conditions: $m_{catalyst}$ = 250 mg; $H_2$/$CO_2$ = 3; P = 20 bar; GHSV = 9917 $h^{-1}$; TOS = 19 h; T = 200-300 °C.

**Fig. 8**        (a) - STY and (b, c) - product selectivity of condensable product collected after gas-phase $CO_2$ hydrogenation over the CuZnFe and xRb/CuZnFe catalysts at different reaction temperatures (200, 250, and 300°C). Reaction conditions: $m_{catalyst}$ = 250 mg; $H_2$/$CO_2$ = 3; P = 20 bar; GHSV = 9917 $h^{-1}$; TOS = 19 h.

**Fig. 9**        Product selectivity - (a) and STY - (b) over the 4%Rb/CuZnFe catalyst in the gas phase. Reaction conditions: $m_{catalyst}$ = 250 mg; $H_2$/$CO_2$ = 3; P = 20 bar; GHSV = 9917 $h^{-1}$; TOS = 19 h; T = 200-300 °C.

**Fig. 10**        (a, b) - product selectivity and STY over the 4%Rb/CuZnFe catalyst in the gas phase; (c, d) - product selectivity and STY over the 4%Rb/CuZnFe catalyst in the liquid phase. Reaction conditions: $m_{catalyst}$ = 250 mg; $H_2$/$CO_2$ = 3; P = 20 bar; GHSV = 9917 $h^{-1}$; TOS = 16 h; T = 300 °C.

**Fig. 11**        $N_2$ adsorption-desorption isotherms - (a), BJH pore size distribution - (b), and XRD patterns - (c,d) of the CuZnFe and xRb/CuZnFe catalysts.

**Fig. 12**        SEM scans of CuZnFe - (a, c) and 4%Rb/CuZnFe samples - (b, d).

**Fig. 13**        STEM images and EDX elemental mappings of CuZnFe - (a) and 4%Rb/CuZnFe - (b) samples.

**Fig. 14**        XPS spectra of (a) - survey, (b) - O 1s, (c) -Zn 2p, (d) - Rb 3d in the CuZnFe and xRb/CuZnFe catalysts.

**Fig. 15**        Deconvoluted O 1s XPS spectra of (a) - CuZnFe, (b) - 2%Rb/CuZnFe, (c) - 4%Rb/CuZnFe, (d) - 8% Rb/CuZnFe and (d) - O 1s distribution.

**Fig. 16.**        The $CO_2$-TPD - (a) and $CO_2$ desorption results - (b) for the CuZnFe and xRb/CuZnFe catalysts.

**Fig. 17.**        *In situ* DRIFT spectra of surface species on the 4%Rb/CuZnFe catalyst during $CO_2$ hydrogenation ($H_2$/$CO_2$ =3/1, P = 20 bar): (a) - spectra at different reaction temperatures; (b, c) - time-resolved spectra at 300 °C.

**Fig. 18**        The scheme and photos of a custom-designed parallel packed bed reactor system

**Fig. 19**        STEM-BF images of CuZnFe - (a) and 4%Rb/CuZnFe - (b) samples.

**Fig. 20**        XPS spectra of (a) - C 1s, (b) - Cu 2p, (c) - Fe 2p in the CuZnFe and xRb/CuZnFe catalysts.

**Fig. 21**        CO selectivity over the CuZnFe and xRb/CuZnFe catalysts in the gas phase. Reaction conditions: $m_{catalyst}$ = 250 mg; $H_2$/$CO_2$ = 3; P = 20 bar; GHSV = 9917 $h^{-1}$; TOS = 19 h; T = 200-300 °C.

**Fig. 22**        Product selectivity over the 4%Rb/CuZnFe catalyst in the liquid phase. Reaction conditions: $m_{catalyst}$ = 250 mg; H2/CO2 = 3; P = 20 bar; GHSV = 9917 $h^{-1}$; TOS = 19 h; T = 200-300 °C.

**Fig. 23**        GC-MS spectrum of liquid-phase products from $CO_2$ hydrogenation over the 4%Rb/CuZnFe catalyst.

**Fig. 24**        Proposed reaction pathways for $CO_2$ hydrogenation over the 4%Rb/CuZnFe catalyst.

**Examples**

**Chemicals used**

Rubidium nitrate (RbNO$_3$, 99%), cupper nitrate (Cu(NO$_3$)$_2$·3H$_2$O, 99%), zinc nitrate (Zn(NO$_3$)$_2$·6H$_2$O, 99%), iron nitrate (Fe(NO$_3$)$_3$·9H$_2$O, 99%), ethanol (C$_2$H$_5$OH, >99.8%), methanol (CH$_3$OH, >99.9%), 1-propanol (C$_3$H$_8$O, 99.5%), 2-propanol (C$_3$H$_8$O, 99.5%), 1-butanol (C$_4$H$_{10}$O, 99.5%), 2-butanol (C$_4$H$_{10}$O, 99.5%), 3-methyl-1-butanol (C$_5$H$_{12}$O, ≥98.5%), 1-pentanol (C$_5$H$_{12}$O, 99.5%), acetone (C$_3$H$_6$O, >99.5%), acetaldehyde (C$_2$H$_4$O, >99.5%), acetic acid (C$_2$H$_4$O$_2$, >99.7%), propanoic acid (C$_3$H$_6$O$_2$, >99%), butanoic acid (C$_4$H$_8$O$_2$, >99%), pentanoic acid (C$_5$H$_{10}$O$_2$, >99.5%), and hexanoic acid (C$_6$H$_{12}$O$_2$, >99%) were purchased from Merck.

**Example 1: Rb-loaded catalyst**

1.1 Catalyst synthesis

*Preparation of CuZnFe catalyst*

CuZnFe catalyst with a molar ratio of Cu:Zn:Fe = 1:1:2 ratio was synthesised from 0.2 M solutions from nitrate powders of Cu(NO$_3$)$_2$·3H$_2$O, Fe(NO$_3$)$_3$·9H$_2$O in Zn(NO$_3$)$_2$·6H$_2$O. Catalyst was prepared by using a coprecipitation method. The solutions were prepared from 0.24 M (NH$_4$)$_2$CO$_3$ and were mixed at 50 °C for 30 min until completely dissolved. The solutions were then mixed together with a single-drop method while vigorously stirred and the temperature was increased to 70 °C and mixed for 1 h. After that the solution was heated to 85 °C and stirred for another 3 h. After that the solution was placed into a dryer at 110 °C overnight. After drying, the catalyst was placed into furnace for calcination at 400 °C for 2 h.

*Preparation of CuZnFe/ZSM-5 catalyst*

H-ZSM-5 commercial zeolite powder (molar SiO$_2$/Al$_2$O$_3$ = 30, Zeolyst Int., CBV 3024E) was pretreated at 550 °C for 6 h in an air followed by cooling down to room temperature before being impregnated. Preparation of the 10wt.% Cu-10wt.%Zn-20wt.%Fe/ZSM-5 catalyst involved the impregnation of zeolite using the wet impregnation method. For a typical synthesis, the experimentally desired amount of Fe(NO$_3$)$_3$·9H2O, Zn(NO$_3$)$_2$·6H$_2$O and Cu(NO$_3$)$_2$·3H$_2$O were dissolved in the corresponding volume of distillate water for obtaining 0.05-0.1 M solutions. The solutions were stirred at 50 °C for 30 min. Next, the solutions of Cu(NO$_3$)$_2$·3H$_2$O were added to the H-ZSM-5 zeolite for 1 h. Thereafter, Zn(NO$_3$)$_2$·6H$_2$O was added to the mixture and stirred for 1 h. Finally, Fe(NO$_3$)$_3$·9H$_2$O was added with vigorous stirring for 1-2 h at 80 °C. After impregnation, all samples were dried overnight at 110°C. Afterward, they were calcined for 6 h at 550°C in an air atmosphere. All catalysts were reduced in flow of H$_2$ for 1 h.

*Preparation of xRb/CuZnFe catalysts*

Catalysts composed of CuZnFe, incorporating varying Rb loadings at 2%, 4%, and 8% by weight, were synthesized through the impregnation of CuZnFe oxides in a 0.01 M aqueous solution of RbNO$_3$. Following impregnation, the samples and the resulting solution underwent a heating process at 70 °C with vigorous stirring, subsequent overnight drying at 110 °C, and final calcination at 400 °C for 2 h. Prior to catalytic testing, all samples were reduced in a hydrogen atmosphere for 1 h to activate the catalysts.

1.2 Catalyst characterization

The textural properties of the catalysts were evaluated using the N$_2$ adsorption-desorption technique on a Micromeritics ASAP 2020 apparatus. Prior to the analysis, a 150 mg sample was degassed overnight at 200 °C (with a temperature ramp of 10 °C/min) under a vacuum of 10$^{-3}$ Pa. The N$_2$ adsorption-desorption measurements were then performed at -196 °C. The pore-size distribution was determined from the desorption isotherms using the Barrett-Joyner-Halenda (BJH) method, while the total surface area was calculated using the Brunauer-Emmett-Teller (BET) method.
The composition of the solid-phase catalysts was determined using powder X-ray diffraction (XRD) analysis conducted on a PANalytical XpertPro instrument. The analysis employed CuK$\alpha$1 radiation (wavelength 1.54056 Å) over a 2θ angle range from 10° to 70°, with a step size of 0.034°. The average crystallite size was estimated using the Scherrer equation, focusing on key diffraction peaks at 33.4°, 35.6°, 36.3°, 38.9°, 54.1°, and 62.9°. The relative crystallinity percentage (RC%) within the 10-70° 2θ range was determined by the formula: RC% = Ic/(Ic + Ia), where Ic corresponds to the total intensity of the crystalline peaks and Ia to the total intensity of the amorphous peaks. Moreover, the RC% was also calculated relative to the CuZnFe catalyst using the equation: RC% = Ic$_{(xRb/CuZnFe)}$/(Ic$_{(CuZnFe)}$ + Ia$_{(CuZnFe)}$), where Ic$_{(xRb/CuZnFe)}$ represents the crystalline intensity of the Rb sample normalized against the combined intensity of the crystalline and amorphous peaks of the CuZnFe catalyst.

The elemental composition was analysed using inductively coupled plasma mass spectrometry (ICP-OES) with an Agilent 7500ce instrument. Prior to analysis, the samples were treated with a mixture of HF and $HClO_4$ to evaporate any volatiles. The remaining residue was then dissolved in a mixture of HCl and $H_3BO_4$, followed by dilution to facilitate the determination of Rb content in the catalyst samples. The morphology of the catalysts was examined using a scanning electron microscope (SEM) (FE-SEM SUPRA 35-F, Carl Zeiss) equipped with an Inca 400 energy-dispersive spectrometer (Oxford Instruments). Particle sizes for each sample were determined from the high-resolution SEM (HRSEM) images using ImageJ software. Additionally, scanning transmission electron microscopy (STEM) micrographs and energy-dispersive X-ray spectroscopy (EDXS) chemical mapping were performed using a JEOL ARM 200 CF microscope, which features a cold field-emission gun and a Jeol Centurio EDXS system.

The X-ray photoelectron spectroscopy (XPS) analyses were carried out on the PHI-TFA XPS spectrometer produced by Physical Electronics Inc equipped with Al-monochromatic source emitting photons at energy of 1486.6 eV. The analyzed area was 0.4 mm in diameter. Quantification of surface composition was performed from XPS peak intensities taking into account relative sensitivity factors provided by instrument manufacturer. Surface sensitivity was 2-5 nm. All data were corrected by the binding energy of C 1s (284.8 eV) adventitious carbon. Accuracy of binding energy was ± 0.3 eV.

$CO_2$ temperature-programmed desorption ($CO_2$-TPD) experiments were performed on a Microtrac MRB BELCAT instrument according to the following procedure: A 100 mg sample underwent reduction under a 5%$H_2$/Ar atmosphere (30 mL/min) at 350 °C for 1 h. Subsequently, the gas flow was switched to He at a rate of 30 mL/min for 1 h to eliminate residual $H_2$, and the system was then cooled to 50 °C. The samples were then subjected to a flow of $CO_2$ (10% $CO_2$ with He as a balance gas) at 50 °C and 30 mL/min for 30 min, after which the gas flow was switched to He at a rate of 30 mL/min for 30 min to remove any remaining $CO_2$. TPD measurements were carried out using He as a carrier gas, ramping the temperature from 50 to 750 °C, with $CO_2$ detected using a thermal conductivity (TCD) detector. Concurrently, the absence of water was confirmed using a quadrupole mass spectrometer detector, specifically targeting the characteristic m/z = 44 fragment, employing an online Pfeiffer Vacuum Thermostar quadrupole mass spectrometer.

In situ diffuse reflectance infrared Fourier transform spectroscopy (DRIFTS) measurements were conducted using a Spectrum 100 FT-IR spectrometer (PerkinElmer) equipped with a mercury cadmium telluride (MCT) detector and a Harrick reaction chamber. Spectra were recorded in the range of 750-4000 $cm^{-1}$ with a spectral resolution of 4 $cm^{-1}$, averaging 8 scans per measurement. Prior to analysis, the sample was reduced at 300 °C under a $H_2$ flow (30 mL/min) for 1 h. Background spectra were collected by averaging 8 scans at a resolution of 4 $cm^{-1}$ after cooling to the target temperature under an $H_2$ flow (30 mL/min). DRIFTS experiments were performed at 25-300 °C under a $H_2/CO_2$ (3:1) gas mixture (30 mL/min) at a total pressure of 20 bar. The sample temperature was controlled by internal thermocouple.

**Results:**

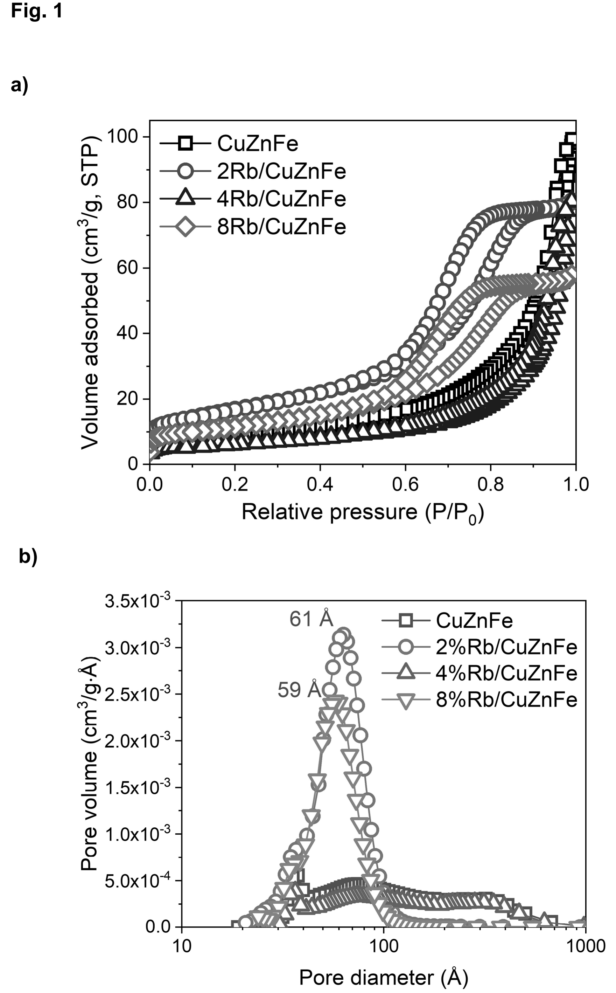

**Fig. 1** shows $N_2$ adsorption-desorption isotherms (a) and BJH pore size distribution (b) of the CuZnFe and xRb/CuZnFe catalysts.

**Table 1**

Elemental composition and structural properties of the CuZnFe and xRb/CuZnFe catalysts.

| Catalyst | Rb[a] (wt %) | Cu[a] (wt %) | Zn[a] (wt %) | Fe[a] (wt %) | $S_{BET}$[b] (m²/g) | $S_{micro}$[b] (m²/g) | $S_{meso}$[b] (m²/g) | $V_{micro}$[c] (cm³/g) | $V_{total}$[c] (cm³/g) | $V_{meso}$[c] (cm³/g) | PD[d] (nm) | Cryst allite size[e] (nm) | RC[e] (%) | RC vs CuZn Fe[e] (%) |
|---|---|---|---|---|---|---|---|---|---|---|---|---|---|---|
| **CuZnFe** | - | 19.0 | 21.0 | 41.0 | 31.7 | 1.9 | 29.8 | $7.6 \cdot 10^{-4}$ | 0.149 | 0.148 | 15.5 | 20.6 | 96.5 | 96.5 |
| **2%Rb/ CuZnFe** | 2.0 | 13.0 | 21.0 | 43.0 | 60.0 | 3.8 | 56.2 | $1.6 \cdot 10^{-3}$ | 0.123 | 0.121 | 6.1 | 18.4 | 79.5 | 60.2 |
| **4%Rb/ CuZnFe** | 3.0 | 18.0 | 19.0 | 38.0 | 23.0 | 4.3 | 18.7 | $2.1 \cdot 10^{-3}$ | 0.119 | 0.197 | 16.6 | 21.3 | 95.9 | 99.6 |
| **8%Rb/ CuZnFe** | 6.0 | 12.0 | 20.0 | 39.0 | 41.2 | 4.7 | 36.5 | $2.2 \cdot 10^{-3}$ | 0.088 | 0.086 | 5.9 | 19.8 | 76.1 | 54.5 |

[a]ICP-OES. [b]BET method. [c]t-plot method. [d]Average pore diameter measured from the desorption branch according to the BJH method. [e]The crystallite size and relative crystallinity (RC) were obtained from XRD data.

Detecting Rb accurately with ICP-OES can be challenging, which could introduce a margin of error in the results. Additionally, there is a possibility that the analyzed portion of the sample may not have been perfectly homogeneous, further contributing to the variation in the measured amounts. However, in SEM-EDX analysis, the results showed a Rb content much closer to the theoretical values, indicating a better correlation with the amounts used during synthesis.

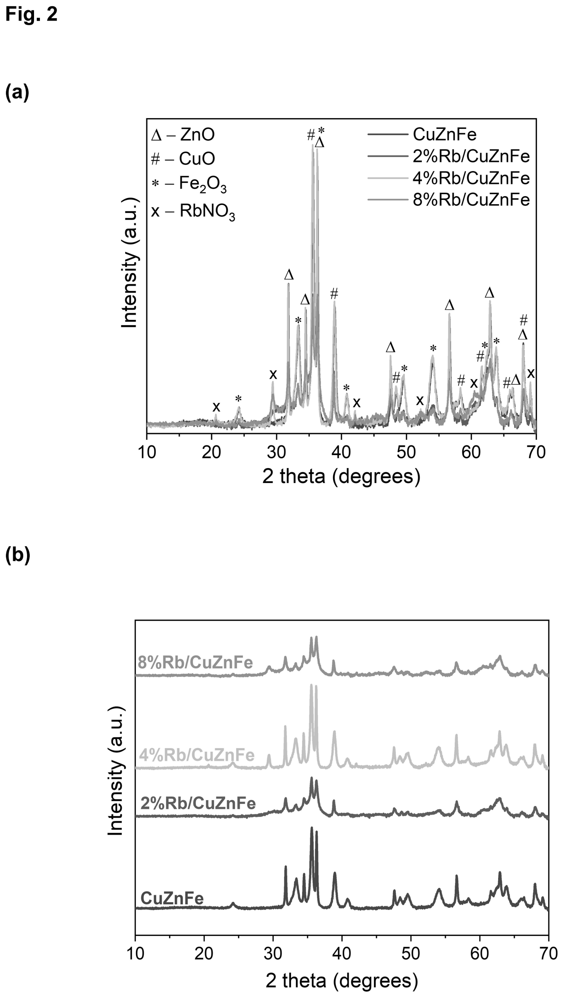

The XRD patterns displayed in **Fig. 2** exhibit the profiles of the CuZnFe and xRb/CuZnFe catalysts. The primary crystalline constituents for the catalysts prior to the reaction encompass ZnO (COD No. 96-900-4182) with peaks at $2\theta$ angles of 31.8°, 34.5°, 36.3°, 47.6°, 56.7°, 62.9°, 66.4°, and 68.0°; CuO (COD No. 96-901-6327) with peaks at $2\theta$ angles of 35.6°, 38.9°, 48.4°, 58.4°, 61.7°, 66.4°, and 68.0°; $Fe_2O_3$ (COD No. 96-154-6384) with peaks at $2\theta$ angles of 24.2°, 33.3°, 35.6°, 40.9°, 49.6°, 54.1°, 62.5°, and 63.9°; as well as $RbNO_3$ (COD No. 96-210-7327) with peaks at $2\theta$ values of 20.7°, 29.5°, 42.2°, 52.3°, 61.3°, 65.4°, and 69.4°.

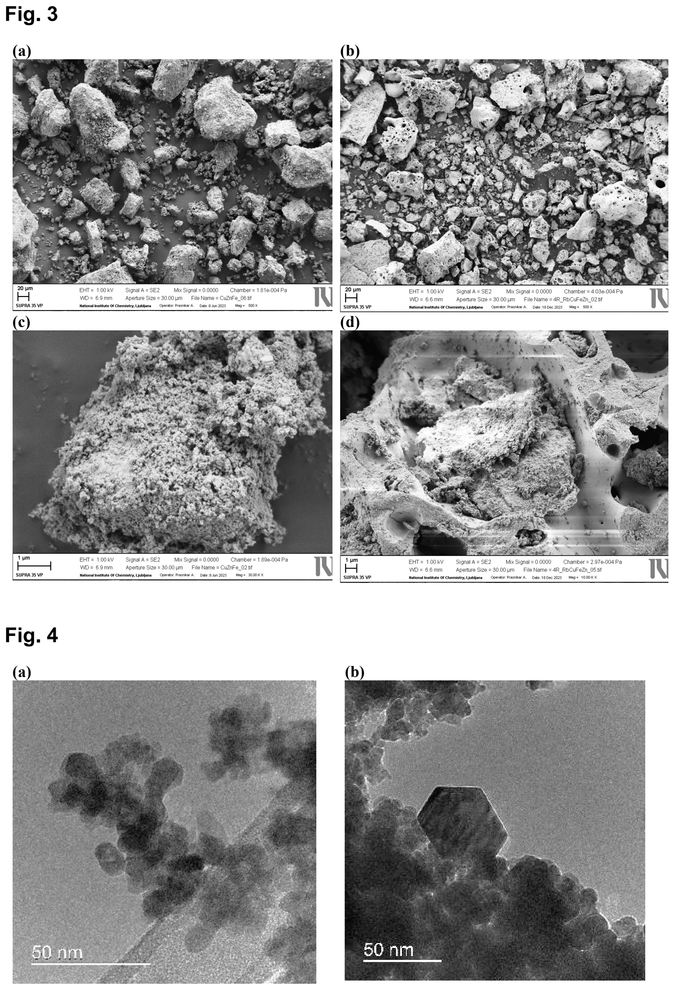

**Fig. 3** shows SEM scans of CuZnFe mixed oxides (a, c) and Rb loaded samples 4%Rb/CuZnFe (b, d).

**Fig. 4** shows STEM-BF images of CuZnFe mixed oxide particles (a) and Rb-loaded samples 4%Rb/CuZnFe - (b).

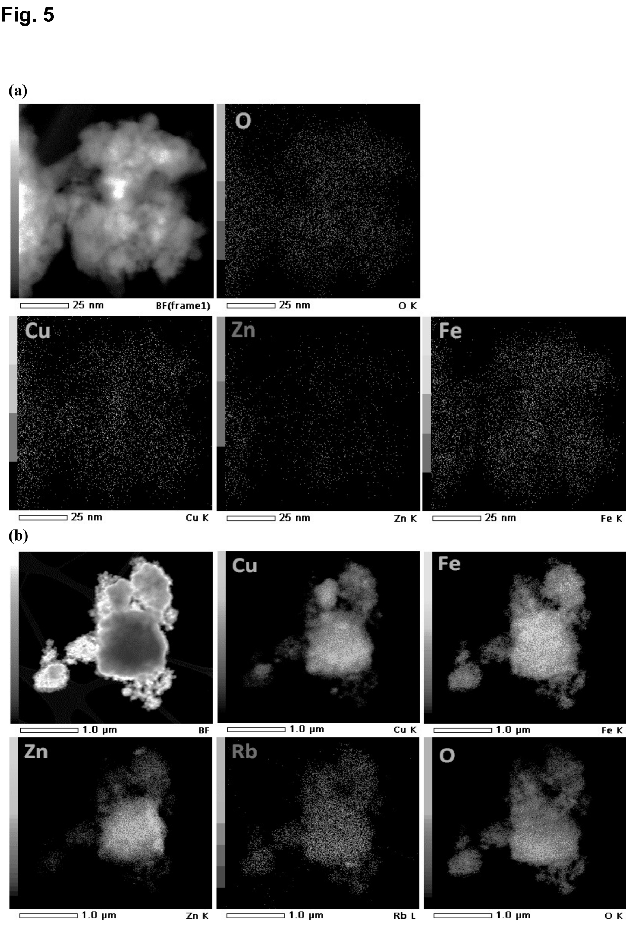

**Fig. 5** shows STEM images and EDX elemental mappings of CuZnFe mixed oxide particles (a) and Rb-loaded samples 4%Rb/CuZnFe - (b).

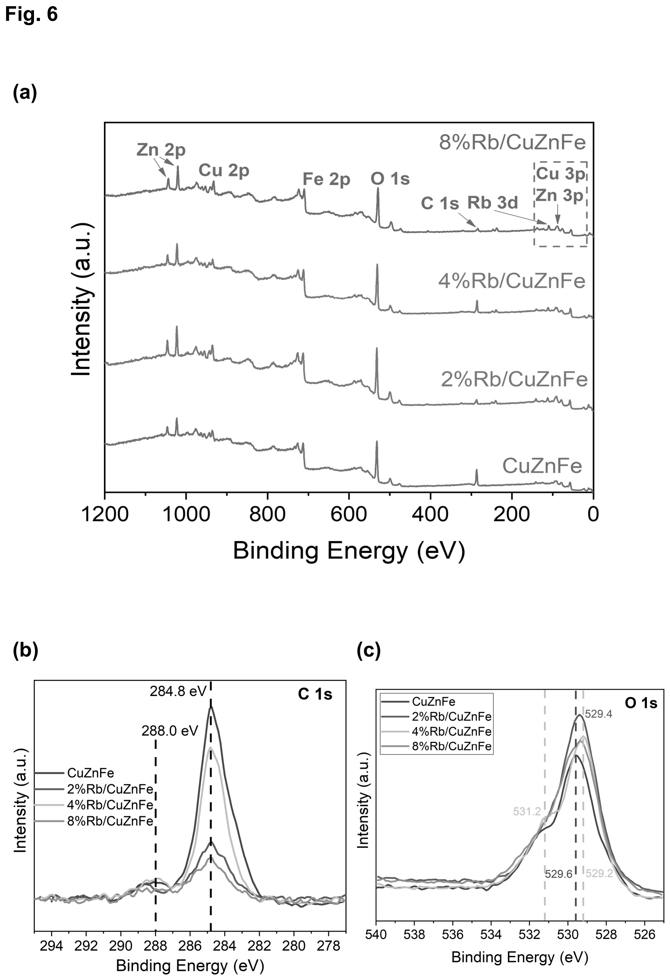

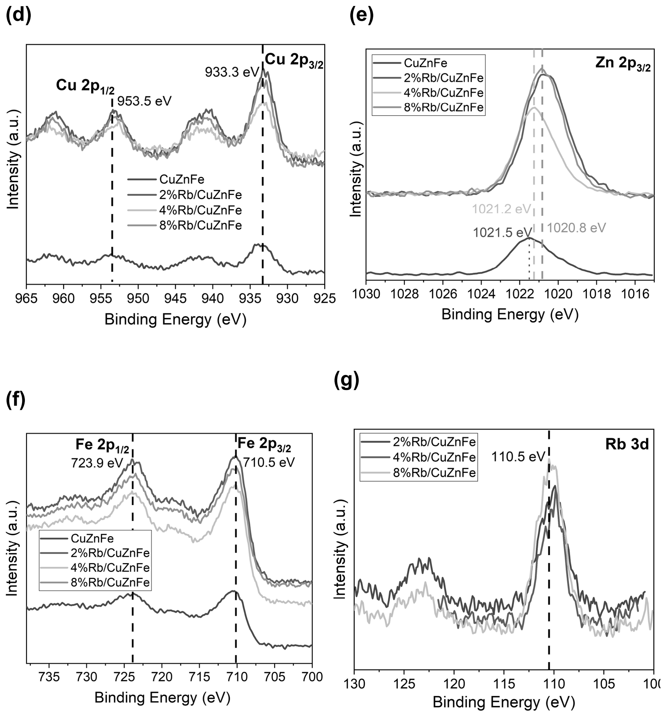

**Fig. 6** shows XPS spectra of (a) - survey, (b) - C 1s, (c) - O 1s, (d) - Cu 2p, (e) -Zn 2p, (f) - Fe 2p, (g) - Rb 3d in the CuZnFe and xRb/CuZnFe catalysts.

### Table 2

| SEM-EDX analysis of the CuZnFe and xRb/CuZnFe catalysts. | | | |
|---|---|---|---|
| **CuZnFe** | | | |
| **Element** | **Average (wt%)** | **Scan 1 (wt%)** | **Scan 2 (wt%)** | **Scan 3 (wt%)** |
| Cu | 20.1 | 20.5 | 20.0 | 19.7 |
| Fe | 35.6 | 35.8 | 35.7 | 35.4 |
| O | 29.7 | 28.9 | 29.7 | 30.5 |
| Zn | 14.6 | 14.8 | 14.6 | 14.4 |
| **2%Rb/CuZnFe** | | | |
| Rb | 2.3 | 2.2 | 2.1 | 2.4 |
| Cu | 15.3 | 16.0 | 15.4 | 14.7 |
| Fe | 40.6 | 40.2 | 41.3 | 40.4 |
| O | 21.0 | 20.5 | 20.8 | 21.8 |
| Zn | 20.8 | 21.1 | 20.5 | 20.7 |
| **4%Rb/CuZnFe** | | | |
| Rb | 1.9 | 2.5 | 1.5 | 1.6 |
| Cu | 20.5 | 20.4 | 20.7 | 20.4 |
| Fe | 36.9 | 36.4 | 37.2 | 37.0 |
| O | 26.1 | 25.8 | 26.1 | 26.5 |
| Zn | 14.6 | 14.9 | 14.4 | 14.5 |
| **8%Rb/CuZnFe** | | | |
| Rb | 4.4 | 4.4 | 4.5 | 4.3 |
| Cu | 13.4 | 13.2 | 13.6 | 13.4 |
| Fe | 38.8 | 38.1 | 38.8 | 39.4 |
| O | 23.9 | 24.4 | 23.7 | 23.8 |
| Zn | 19.5 | 19.9 | 19.5 | 19.2 |

**Table 3**

| Surface composition of CuZnFe and xRb/CuZnFe catalysts as determined by XPS analysis. | | | | | | |
|---|---|---|---|---|---|---|
| **Catalyst** | **C 1s, at.%** | **O 1s, at.%** | **Fe 2p, at.%** | **Cu 2p, at.%** | **Zn 2p, at.%** | **Rb 3d, at.%** |
| CuZnFe | 37.4 | 44.5 | 8.2 | 5.0 | 4.8 | 0.0 |
| 2%Rb/CuZnFe | 13.2 | 56.0 | 10.2 | 9.8 | 8.6 | 2.2 |
| 4%Rb/CuZnFe | 28.4 | 51.0 | 8.8 | 4.8 | 5.1 | 1.8 |
| 8%Rb/CuZnFe | 10.2 | 58.2 | 10.2 | 97 | 8.5 | 3.3 |

1.3 <u>Catalytic performance</u>

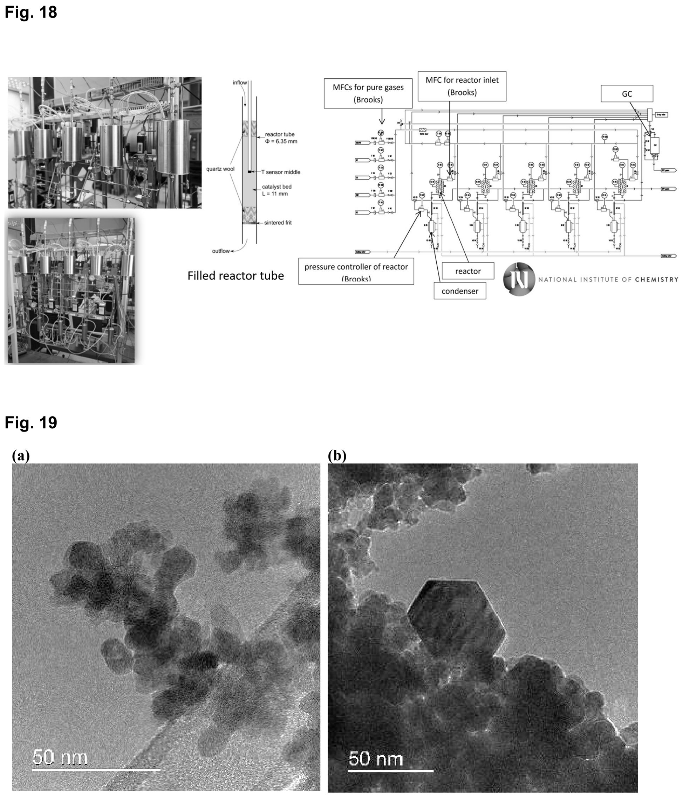

The catalysts were assessed in their powdered form (0.25 g), loaded into a parallel packed bed reactor unit with quartz wool, utilizing a 6.35 mm tube **(Fig. 18).** A gas chromatograph equipped with a TCD detector and CP-Molsieve and PoraPlot U column was connected to the gas outlet of the reactor. The gases employed for testing and reduction were hydrogen ($H_2$) with a purity of 5.0 (Messer) and carbon dioxide ($CO_2$) with a purity of 4.5 (Messer), mixed in a ratio of $H_2/CO_2 = 3$. Prior to the reaction, the catalyst was subjected to reduction in hydrogen gas at atmospheric pressure and 300 °C for 1 h.

The gas reaction mixture was supplied through hydrogen and carbon dioxide cylinders, mixed before entering the reactor. The gas feed rate was maintained at GHSV = 9917 $h^{-1}$, and the reactor feed rate was set at (0.03 g/min or 60 ml/min), while the reaction was carried out at a pressure of 20 bar. The temperature program for the catalytic testing in the reactor consisted of 14 holding the temperature at 200 °C for 8 h, increasing it to 250 °C for 3 h, and then raising it further to 300 °C for 5 h, with 30 min of heating between temperature ramps.

All reactions were performed in the gas phase. The term 'liquid products' or 'liquid-phase' refers to condensable oxygenates collected from the product stream downstream in a cold trap. Condensable liquid products (primarily oxygenates) were collected downstream in a cold trap after each catalytic run (typically after 19 h on stream). These products originated from the gas-phase $CO_2$ hydrogenation process. The total volume of collected liquid products was typically between 1.5 and 2.0 mL per run. The liquid products obtained were analysed offline using a gas chromatography-mass spectrometry system, specifically the Agilent GC-7890A combined with the Agilent 5977B GC/MSD. This system was equipped with a DB-WAX Ultra Inert capillary column, measuring 30 m in length, having an internal diameter of 0.25 mm, and featuring a film thickness of 0.25 $\mu$m. The identification and quantification of these liquid products were achieved through an external calibration technique, spanning the 0.01-5 wt% range and employing the chemicals detailed in the chemical section.

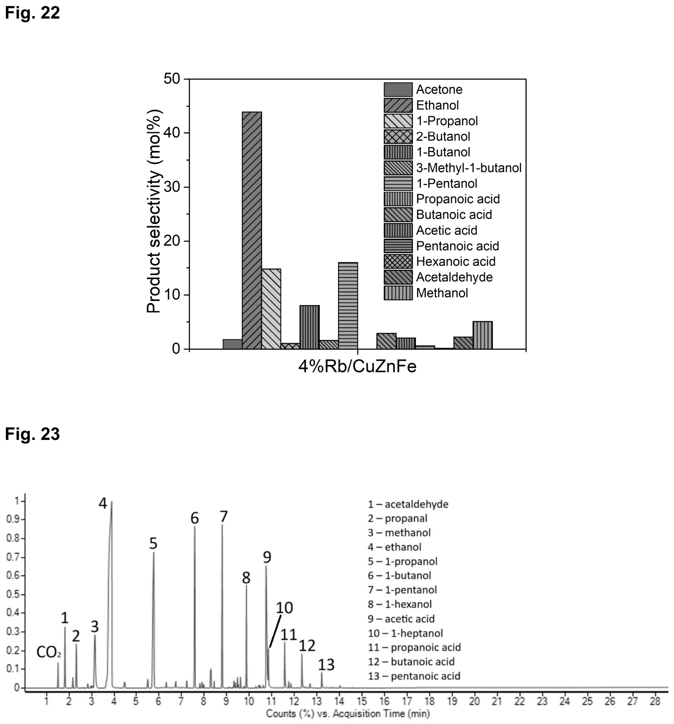

The primary liquid products identified included EtOH, 1-propanol, 1-pentanol, 1-butanol, and acetic acid **(Fig. 23).** The GC-MS analysis also detected traces of liquid byproducts, including acetone, 2-butanol, 3-methyl-1-butanol, propanoic acid, butanoic acid, acetic acid, pentanoic acid, hexanoic acid, and acetaldehyde. The relative errors in the measured product concentrations were determined to be less than 5%. The carbon balance consistently exceeded 95%.

*Calculation details of catalytic performances*

The total conversion of $CO_2$ ($X_{CO2}$), was calculated using the next equation:

$$X_{CO2}(mol\%) = \frac{n(CO2)_{in} - n(CO2)_{out}}{n(CO2)_{in}} \times 100\% \qquad (1)$$

where $n(CO_2)_{in}$ and $n(CO_2)_{out}$ are the input and output moles of $CO_2$ before and after the reaction, respectively.
The carbon product selectivity, $S(C_i)$, (excluding CO fraction) was calculated using the equation below:

$$S(C_i)(mol\%) = \frac{mol(P_i) \times C_n}{\sum mol(P_i) \times C_n - n(CO)out} \times 100\% \qquad (2)$$

$n(P_i)$ and $C_n$ are specified reaction product and carbon number, respectively. The values in this article are in mol percent.
While CO fraction was calculated accordingly:

$$S_{CO}(mol\%) = \frac{n(CO)_{out}}{\sum mol(P_i) \times C_n} \times 100\% \qquad (3)$$

The total mass balance was always higher than 98%. The space time yield (STY) was calculated as following equation (3-4), where the $n_i$ and $m_i$ represent the number moles or mass of product (EtOH and/or $C_{2+}$ alcohols), $m_{cat}$ represents the mass of catalysts (g), t represents the reaction time (h).

$$\text{STY} \left( \text{mmol} \cdot \text{g}_{cat}^{-1} \cdot \text{h}^{-1} \right) = \frac{n_i}{(m_{cat} \cdot t)} \cdot 1000 \tag{4}$$

Gas hourly space velocity (GHSV) were calculated as follow, where FR is the volumetric flow rate of the gas mixture of $H_2$ and $CO_2$ at STP, ml/h; and $V_{cat}$ is the volume of the catalyst bed, ml.

$$\text{GHSV}_{total} \left( \text{h}^{-1} \right) = \frac{FR\left(\frac{mL}{h}\right)}{V_{cat}(mL)} \tag{5}$$

The carbon balance (CB) was calculated according to the following:

$$\text{CB} = \frac{n(CO2)_{out} + n(CO)_{out} + \sum mol(P_i) \times C_n}{n(CO2)_{in}} \tag{6}$$

**Results:**

*The effect of Rb loading, ZSM-5 support, reaction time and temperature on the composition of the gas product*

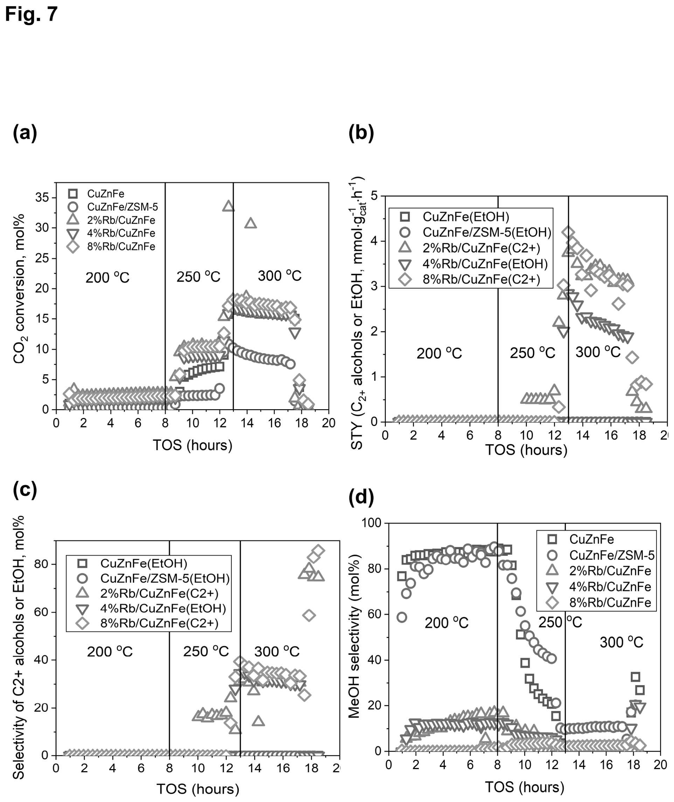

The catalytic performance of CuZnFe and xRb/CuZnFe catalysts in the hydrogenation of $CO_2$ was evaluated in terms of $CO_2$ conversion **(Fig. 7a)**, STY of $C_{2+}$ alcohols or EtOH **(Fig. 7b)**, selectivity towards $C_{2+}$ alcohols and/or EtOH **(Fig. 7c)**, and MeOH selectivity **(Fig. 7d)** in the gas phase. As shown in **Fig. 7a**, at 200 °C, all catalysts exhibited low $CO_2$ conversion, ranging from ~1 to 3%. The unmodified CuZnFe and CuZnFe/ZSM-5 catalysts showed minimal activity, whereas Rb-modified CuZnFe catalysts demonstrated slightly higher conversion. Increasing the temperature to 250 °C resulted in a noticeable enhancement in $CO_2$ conversion, reaching 4-7% for CuZnFe and CuZnFe/ZSM-5, respectively. Notably, the xRb/CuZnFe catalysts displayed higher conversions, approximately 9-11%. At 300 °C, the $CO_2$ conversion reached its peak, with xRb/CuZnFe attaining the highest value of ~17-19%. These results suggest that Rb incorporation improves $CO_2$ activation and hydrogenation, likely due to electronic modifications and improved catalyst stability.

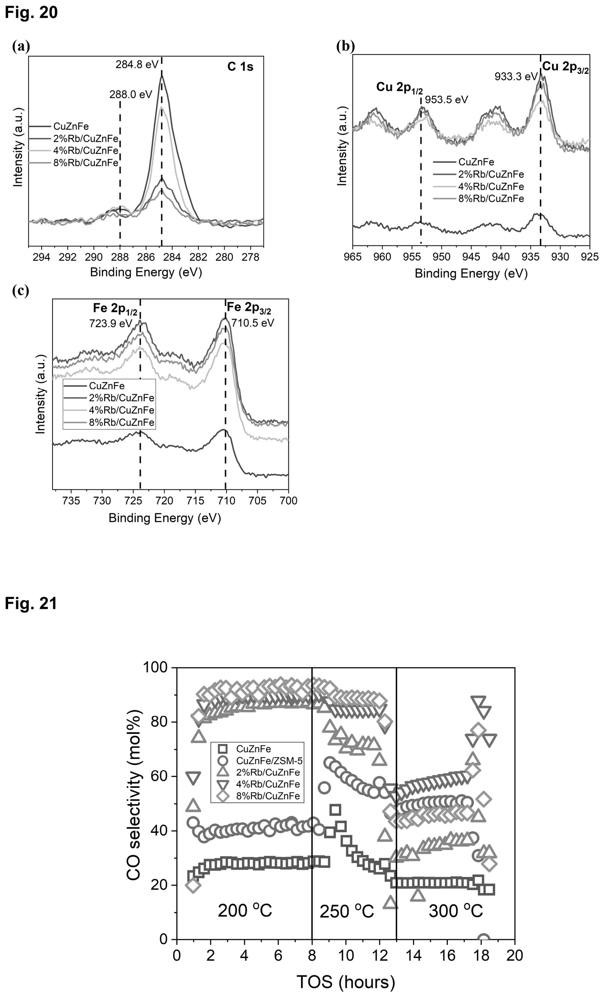

At 200 °C, the STY of $C_{2+}$ alcohols, including ethanol, was negligible for all catalysts investigated. Upon increasing the reaction temperature to 250 °C, a significant rise in STY was observed for the Rb-modified catalysts, particularly for 2%Rb/CuZnFe, which reached ~0.5 mmol·g$^{-1}$·h$^{-1}$. Further increasing the temperature to 300 °C led to a substantial enhancement in the STY of $C_{2+}$ alcohols, with 8%Rb/CuZnFe exhibiting the highest value (~4.2 mmol·g$^{-1}$·h$^{-1}$), followed by 2%Rb/CuZnFe (~3.7 mmol·g$^{-1}$·h$^{-1}$). Notably, the 4%Rb/CuZnFe catalyst demonstrated the highest STY specifically for EtOH as the main product, reaching 2.8 mmo·g$^{-1}$·h$^{-1}$. In contrast, no formation of $C_{2+}$ alcohols was observed over the CuZnFe and CuZnFe/ZSM-5 catalysts, suggesting that the unpromoted CuZnFe catalyst and the zeolite-modified system primarily favored methanol formation over HA synthesis. The STY trends suggest that Rb incorporation plays a crucial role in enhancing $C_{2+}$ alcohol production by promoting C-C coupling over Cu-based catalysts. The selectivity trends in **Fig. 7c** reveal that at 200 °C, all catalysts predominantly formed MeOH, without formation towards HA. As the temperature increased to 250 °C, the selectivity towards $C_{2+}$ alcohols improved for Rb-modified catalysts, particularly for 2%Rb/CuZnFe (~18%). At 300 °C, the highest selectivity toward $C_{2+}$ alcohols was achieved with the 8% Rb/CuZnFe catalyst, reaching ~86%, followed by 2%Rb/CuZnFe (~78%). In contrast, the 4%Rb/CuZnFe catalyst exhibited the highest selectivity toward ethanol (35%), whereas no formation of higher alcohols was observed over the CuZnFe and CuZnFe/ZSM-5 catalysts. The methanol selectivity trends **(Fig. 7d)** illustrate the differences between the CuZnFe, CuZnFe/ZSM-5, and Rb-modified CuZnFe catalysts. At 200 °C, the CuZnFe and CuZnFe/ZSM-5 catalysts exhibited similar MeOH selectivity, reaching a maximum of ~88%. In contrast, the Rb-modified catalysts demonstrated significantly lower MeOH selectivity (12-16%), with no detectable amounts observed for the 8%Rb/CuZnFe catalyst, indicating a shift toward HA formation. As the reaction temperature increased to 250 °C, MeOH selectivity further declined for the Rb-modified catalysts (3-7%). At 300 °C, methanol selectivity reached its lowest values, with ~2% observed for the 8%Rb/CuZnFe catalyst and no detectable MeOH for the 2%Rb/CuZnFe catalyst, confirming that Rb promotion effectively redirects selectivity from MeOH toward HA synthesis. **Fig. 21** illustrates the CO selectivity over CuZnFe and xRb/CuZnFe catalysts under varying reaction temperatures and TOS. The results indicate that the unmodified CuZnFe catalyst exhibits the lowest CO selectivity, remaining around 20-30% throughout the experiment, with a slight decrease as the reaction temperature increases.

The introduction of ZSM-5 into the CuZnFe system leads to an increase in CO selectivity to approximately 40-60%, although selectivity fluctuates, particularly at higher temperatures. The incorporation of Rb into the CuZnFe catalyst significantly enhances CO selectivity, with higher Rb loadings leading to more pronounced effects. At 200 °C, the CO selectivity of Rb-modified catalysts is already notably higher than that of the unmodified CuZnFe system. As the temperature increases to 250 °C and 300 °C, CO selectivity continues to rise, particularly for catalysts containing 4%Rb and 8%Rb, which maintain selectivity levels above 80-90% at higher temperatures. Thus, the incorporation of Rb into CuZnFe catalysts had a profound impact on $CO_2$ hydrogenation performance, resulting in enhanced $CO_2$ conversion, increased STY of $C_{2+}$ alcohols, and improved selectivity toward EtOH and other HA products. At 300 °C, the 8% Rb/CuZnFe catalyst exhibited the highest $CO_2$ conversion, STY, and selectivity toward $C_{2+}$ alcohols, while the highest STY and selectivity for EtOH were achieved with the 4%Rb/CuZnFe catalyst. In contrast, the CuZnFe and CuZnFe/ZSM-5 catalysts remained highly selective for MeOH. These findings indicate that Rb promotion facilitates C-C coupling, thereby suppressing MeOH formation and promoting the synthesis of HA.

The incorporation of alkali metals often leads to complex, non-linear changes in catalyst behaviour due to simultaneous and competing influences on the electronic properties, dispersion of active phases, and surface acid-base characteristics. The selectivity does not follow a strictly monotonic trend with increasing Rb loading, likely due to the ability of different Rb concentrations to stabilize distinct surface intermediates or promote competing reaction pathways. Characterization data (BET, XPS, DRIFTS) indicate that the 4%Rb/CuZnFe catalyst provides a favourable balance of surface oxygen vacancies, preserved crystallinity, and high Cu dispersion. This optimal combination promotes EtOH formation over deeper chain growth or methanation. At 2% or 8%Rb, either insufficient electronic modulation (at low Rb) or excessive surface restructuring/blocking (at high Rb) may shift the pathway toward more $C_{2+}$ alcohols or reduce selectivity altogether. As confirmed by ICP-OES and SEM-EDX analyses (see below), the actual surface concentrations of Rb do not necessarily correlate with the nominal loadings, likely due to surface enrichment or leaching phenomena occurring during the synthesis process. Thus, the catalytic behaviour reflects the effective surface composition rather than the bulk content, which can also help explain the apparent anomaly of the 4%Rb sample being more ethanol-selective than its neighbours.

*The effect of Rb loading on the composition of the liquid product and STY*

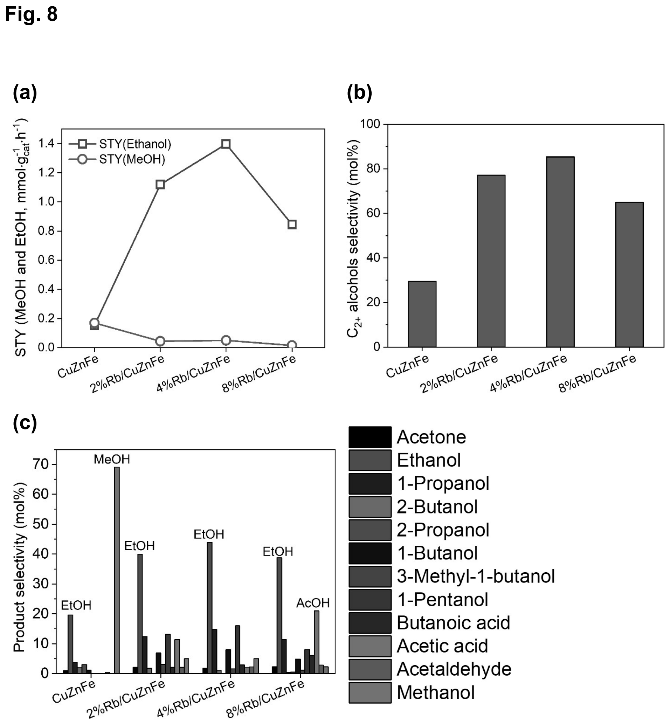

The catalytic performance of CuZnFe and Rb-promoted CuZnFe catalysts was evaluated for the selective production of EtOH and MeOH via gas-phase $CO_2$ hydrogenation, with the resulting condensable liquid products (oxygenates) analysed as shown in **Fig. 8.** The STY of both products demonstrated a clear dependence on the Rb loading. The unmodified CuZnFe catalyst showed minimal activity for both EtOH and MeOH formation, with STY values around 0.15 $mmol \cdot g_{cat}^{-1} \cdot h^{-1}$. This low activity highlights the necessity of promoter modification to enhance the catalytic performance. Notably, no liquid products were detected for the CuZnFe/ZSM-5 catalyst, indicating its limited activity toward the formation of condensable oxygenates under the investigated conditions. Upon introducing Rb as a promoter, a significant enhancement in EtOH production was observed, with the 4%Rb/CuZnFe catalyst achieving the highest ethanol STY of approximately 1.4 $mmol \cdot g_{cat}^{-1} \cdot h^{-1}$. This remarkable improvement can be attributed to the synergistic effects of Rb on the active sites, which likely improved $CO_2$ activation and the subsequent C-C coupling steps necessary for ethanol formation. In contrast, the methanol STY exhibited a marginal decrease across the series, with a maximum value of approximately 0.17 $mmol \cdot g_{cat}^{-1} \cdot h^{-1}$ on the CuZnFe catalyst.

This indicates that the presence of Rb favours EtOH production over MeOH, shifting the product distribution. Interestingly, further increasing the Rb loading to 8% resulted in a decrease in the STY of EtOH, suggesting that excessive Rb may block active sites or alter the catalyst surface properties detrimentally, reducing its overall efficiency. This trend underscores the importance of optimizing promoter concentration to achieve the desired selectivity and activity. The selective enhancement of EtOH over MeOH with Rb promotion can be attributed to the modification of the electronic and geometric properties of the catalyst surface. Rb likely influences the adsorption strength of $CO_2$-derived intermediates and stabilizes $C_2$ intermediates, promoting EtOH formation. Additionally, Rb role in suppressing MeOH formation pathways may contribute to the observed selectivity shift. These findings highlight the critical role of promoter optimization in tailoring the catalytic performance of CuZnFe-based systems for $CO_2$ hydrogenation.

The product selectivity results for $CO_2$ hydrogenation over CuZnFe and Rb-modified CuZnFe catalysts provide valuable insights into the influence of Rb on the reaction pathways and product distribution **(Figs. 8b-c).** The selectivity trends reveal the diverse range of oxygenates formed, including methanol, ethanol, 1-propanol, 2-butanol, 2-propanol, 1-butanol, 3-methyl-1-butanol, 1-pentanol, butanoic acid, acetic acid, and acetaldehyde. The unmodified CuZnFe catalyst exhibited a high selectivity toward MeOH (~70%), with EtOH selectivity remaining below 20%. This indicates that, without Rb promotion, the primary pathway favored single-carbon products, reflecting the poor ability of CuZnFe to catalyze C-C coupling reactions. The introduction of Rb dramatically altered the product distribution. For the 2%Rb/CuZnFe catalyst, the methanol selectivity decreased sharply to below 5%, accompanied by a significant increase in EtOH selectivity ~40%. This shift suggests that Rb facilitates C-C coupling reactions and stabilizes intermediates conducive to EtOH formation. Additionally, a modest increase in selectivity toward HA such as propanol, butanol, and pentanol was observed, indicating the catalyst improved ability to promote chain growth.

The 4%Rb/CuZnFe catalyst exhibited high selectivity for EtOH (~44%) and $C_{2+}$ alcohols (85.3%), while maintaining low MeOH selectivity (below 5%). Notably, the production of propanol, butanol, and especially pentanol increased, indicating an enhanced capacity for C-C coupling. However, increasing the Rb loading to 8% resulted in a decrease in EtOH selectivity (~38%) and $C_{2+}$ alcohols (~65%), accompanied by a significant rise in acetic acid production. This suggests that higher Rb loading promotes secondary oxidation pathways, leading to the partial overoxidation of intermediates under certain conditions. This trend aligns with the observed decrease in STY, highlighting the adverse effects of excessive Rb loading, possibly due to overcoverage of active sites or alterations in electronic properties that shift the balance of reaction pathways. Overall, these results demonstrate that Rb promotion not only shifts selectivity from MeOH to EtOH but also opens up new reaction pathways leading to higher oxygenates and byproducts. The optimal Rb loading of 4% achieves the highest EtOH selectivity with minimal MeOH formation, while excessive Rb loading promotes side reactions, highlighting the importance of fine-tuning promoter concentration for desired catalytic performance.

*The product distribution over 4%Rb/CuZnFe catalyst at different reaction temperature in the gas phase*

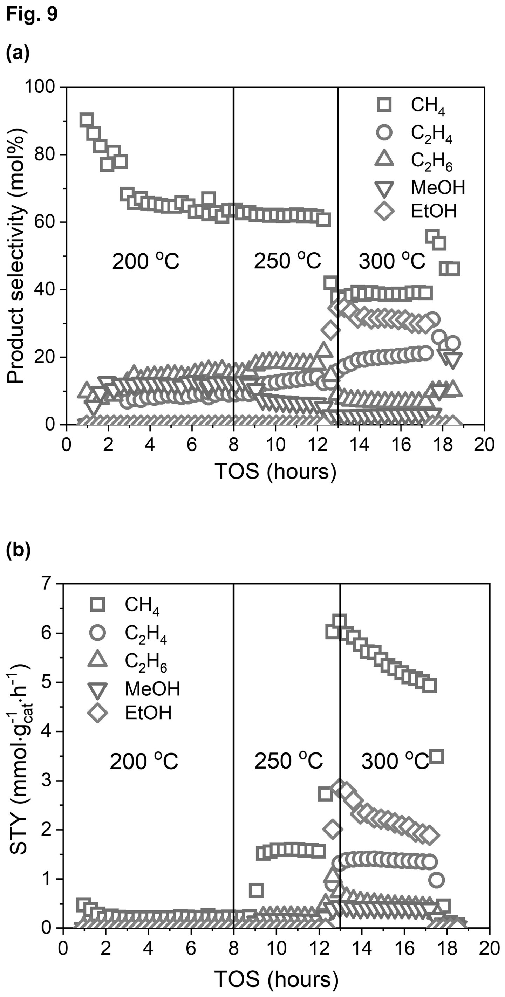

The results presented in **Fig. 9** illustrate the profound impact of reaction temperature on the product selectivity and STY of various products over the most active 4%Rb/CuZnFe catalyst in the gas phase under 20 bar. The observed trends provide key insights into the catalytic performance and reaction pathways involved in $CO_2$ hydrogenation. At 200 °C, $CH_4$ formation dominates the product distribution, with an initial selectivity exceeding 90%, which gradually stabilizes around 65% after prolonged TOS **(Fig. 9a)**. The high $CH_4$ selectivity at this temperature suggests that methanation pathways are highly favoured under these conditions, likely due to the low activation energy required for $CO_2$ conversion to $CH_4$. The presence of minor quantities of higher hydrocarbons such as ethylene (~8%) and ethane (~16%) indicates limited C-C coupling at this temperature. Additionally, MeOH (~12%) and EtOH (~1%) appear in trace amounts, suggesting that oxygenate formation is not significantly promoted at low reaction temperatures. The relatively low STY of all products at this stage further supports the limited catalytic activity and selectivity for EtOH synthesis.

A significant shift in selectivity is observed when the temperature is increased to 250 °C. $CH_4$ selectivity declines, while the formation of ethylene (~12%) and ethane (~20%) increases, implying an enhancement in C-C bond formation and the suppression of complete hydrogenation pathways. The decreasing of MeOH and the same low amount of EtOH suggest a shift in reaction selectivity away from MeOH production but without a substantial improvement in EtOH formation. These changes can be attributed to an increase in the rate of C-C coupling reactions, which facilitate hydrocarbon growth, while EtOH formation remains kinetically limited under these conditions. Additionally, the enhanced catalytic activity may promote competing pathways, reducing MeOH formation while redirecting intermediates toward hydrocarbon products.

At 300 °C, further modifications in product selectivity are evident. $CH_4$ selectivity decreases below 40%, while EtOH selectivity reaches approximately 35%, with an STY of 2.8 $mmol \cdot g_{cat}^{-1} \cdot h^{-1}$, marking a substantial enhancement in EtOH production **(Fig. 1b)**. Additionally, ethylene selectivity increases to 21%, with an STY of ~1.4 $mmol \cdot g_{cat}^{-1} \cdot h^{-1}$, indicating a pronounced shift toward hydrocarbon formation. Interestingly, ethane selectivity decreases to (~8%), suggesting that higher temperatures favour dehydrogenation pathways leading to olefins rather than full hydrogenation to alkanes. The increase in STY for all products at 300 °C confirms that higher reaction temperatures enhance overall catalytic activity. However, fluctuations in product selectivity at extended TOS indicate potential stability issues, which may arise due to catalyst deactivation caused by sintering, coking, or changes in the active site structure.

Overall, these findings demonstrate that the 4%Rb/CuZnFe catalyst exhibits a strong tendency for $CH_4$ formation at lower temperatures, whereas higher temperatures promote the formation of EtOH and $C_2H_4$, shifting the reaction toward more valuable oxygenates and hydrocarbons. The observed temperature-dependent product distribution underscores the importance of fine-tuning reaction conditions to optimize EtOH yield while minimizing unwanted $CH_4$ formation.

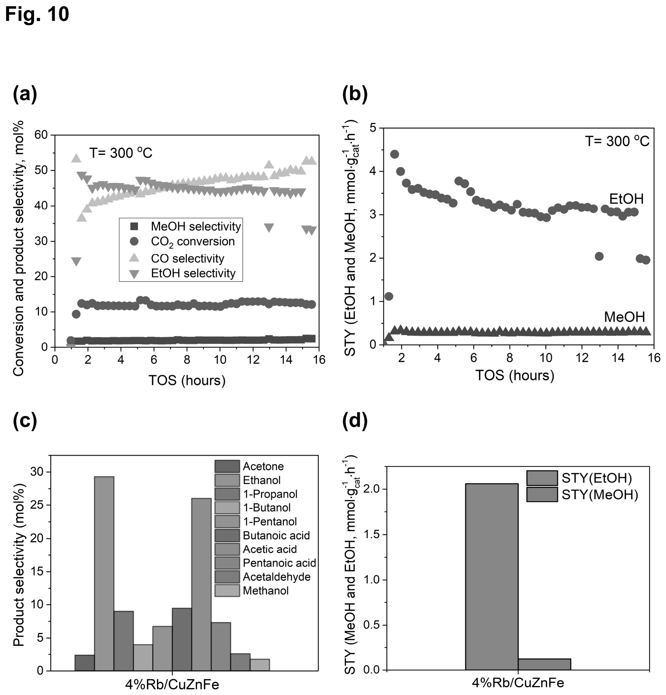

The catalytic performance of the 4%Rb/CuZnFe catalyst for $CO_2$ hydrogenation was evaluated based on both gas-phase and condensable (liquid-phase) product distributions obtained under gas-phase reaction conditions. The selectivity and STY of EtOH and MeOH were analysed as a function of TOS over 16 h. The gas-phase catalytic results **(Fig. 10a)** indicate a stable $CO_2$ conversion of approximately 12% over the 16 h reaction period. Ethanol selectivity remained relatively high (~45%) while CO selectivity was also significant (~40-50%), suggesting that the reverse water-gas shift (RWGS) reaction is a major competing pathway. Methanol selectivity was consistently low (<2%), indicating that under these conditions, the catalyst favours $C_2$ oxygenates over $C_1$ products. The STY profiles in **Fig. 10b** further support these findings, showing a high initial STY of EtOH (~4.4 $mmol \cdot g_{cat}^{-1} \cdot h^{-1}$), which gradually declines but remains stable around ~3.0 $mmol.g_{cat}^{-1} \cdot h^{-1}$ during 16 h. In contrast, the STY of MeOH is negligible (<0.3 $mmol.g_{cat}^{-1} \cdot h^{-1}$) throughout the reaction duration, reinforcing the catalyst preferential selectivity toward EtOH.

In the condensable (liquid-phase) product fraction **(Fig. 10c** and **Fig. 22),** EtOH was the dominant product across different reaction conditions, reaching the highest selectivity (~30-44%), followed by HA, including 1-propanol (~9-14%), 1-butanol (~4-7%), and 1-pentanol (~7-16%). These results suggest that the catalyst facilitates C-C coupling, leading to the formation of higher oxygenates. The presence of acetic acid, butanoic acid, and pentanoic acid in the condensable (liquid-phase) products indicates possible secondary oxidation or condensation reactions during $CO_2$ hydrogenation.

The increased acetic acid selectivity observed in **Fig. 10c** is likely attributed to the immediate exposure of the fresh catalyst to a higher initial temperature, which may influence the formation and evolution of surface intermediates, favor alternative oxidation pathways, and induce distinct deactivation mechanisms compared to the gradual temperature ramp applied in **Fig. 8c.** Such differences in thermal treatment and TOS can modify the distribution and oxidation state of active sites, ultimately altering the product selectivity.

The relatively low MeOH selectivity (<2%) aligns with the gas-phase results, further confirming the catalyst preference for EtOH and HA over MeOH. The STY values in **Fig. 10d** show that EtOH has a much higher STY compared to MeOH in the condensable (liquid-phase) products, further emphasizing the selectivity of the 4%Rb/CuZnFe catalyst toward EtOH production. This could be attributed to the electronic and structural properties of the Rb-modified CuZnFe catalyst, which may enhance the stabilization of key $C_2$ intermediates while suppressing excessive hydrogenation to MeOH. The detection of HA in the condensable (liquid-phase) products indicates that subsequent chain growth via CO insertion or aldol-type condensation mechanisms is also taking place. Additionally, the presence of acids suggests that some intermediates may undergo oxidation under reaction conditions. This could be a competing pathway where acetaldehyde, ethanol, or HA partially oxidize to acetic acid or other carboxylic acids, depending on the catalyst basicity and redox properties.

### 1.4 Catalyst characterization

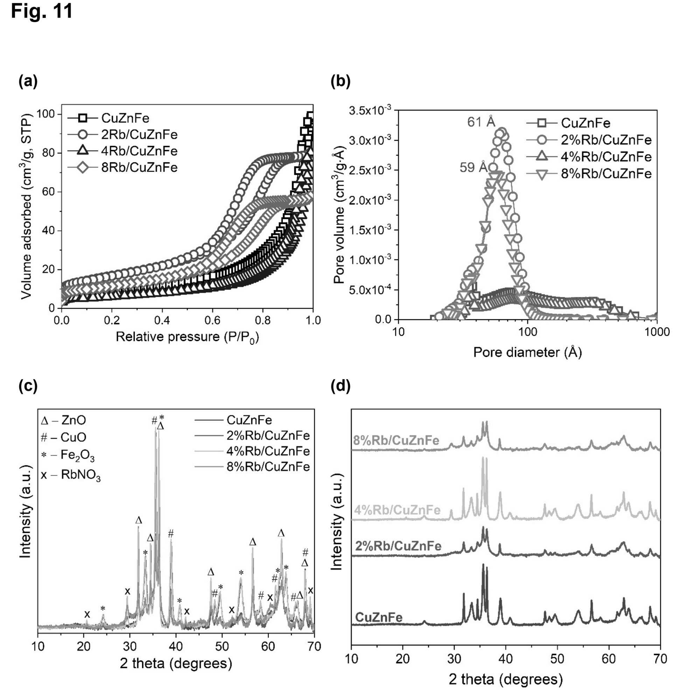

**Fig. 11a** shows the $N_2$ adsorption-desorption isotherms of the CuZnFe and xRb/CuZnFe catalysts. All samples display type IV isotherms with hysteresis loops classified as type $H_3$, which are characteristic of mesoporous materials. Among the catalysts, the 2%Rb/CuZnFe sample exhibits the highest $N_2$ adsorption volume, indicating a significant increase in surface area and porosity compared to the undoped CuZnFe catalyst. The increase in adsorption for the 2% Rb/CuZnFe catalyst suggests that the introduction of Rb at this level effectively enhances the surface area, porosity or mesostructured formation providing more accessible active sites for catalysis. The 8%Rb/CuZnFe catalyst also shows an increase in adsorption, though it is lower than that of the 2%Rb/CuZnFe sample possibly due to pore blocking or collapse of mesoporous structure. In contrast, the 4%Rb/CuZnFe catalyst displays a decrease in $N_2$ adsorption compared to CuZnFe, despite being the most catalytically active. The BJH pore size distribution curves **(Fig. 11b)** provide further insight into the textural characteristics of the catalysts. The pore size calculated using the BJH equation from the desorption branch shows that the 2%Rb/CuZnFe and 8%Rb/CuZnFe catalysts have a narrower pore size distribution, ranging from 20 to 100 nm, with peaks at 61 Å and 59 Å, respectively.

In contrast, the CuZnFe and 4%Rb/CuZnFe catalysts display broader pore size distributions, extending from 20 to 1000 nm, indicating the presence of a wider range of pore sizes. The CuZnFe catalyst has a peak pore diameter (PD) of approximately 35 Å, indicating a broad distribution of pore sizes that contributes to its overall porosity. The 4%Rb/CuZnFe catalyst exhibits a similar peak PD around 35 Å. However, despite having a comparable broad pore size distribution, the 4%Rb/CuZnFe catalyst demonstrates significantly superior catalytic performance. This suggests that the pore structure in the 4%Rb/CuZnFe catalyst, while broad, is optimized for catalytic activity, potentially offering a balance between active site exposure and reactant diffusion.

The elemental compositions of the catalyst samples, including the Cu, Zn, Fe, and Rb contents, were analysed using ICP-OES **(Table 1).** It was revealed an interesting trend in Cu content across the Rb-modified CuZnFe catalysts. While increasing Rb loading generally correlates with a reduction in Cu content, likely due to leaching, an exception was observed in the 4%Rb/CuZnFe sample, which maintained a high Cu content of 18 wt%. This result suggests that an optimal Rb loading of around 4 wt% might enhance the stability of Cu within the catalyst matrix. The relatively high Cu content in the 4%Rb/CuZnFe sample could be attributed to a stabilizing effect provided by the moderate addition of Rb. At this specific loading, Rb may occupy positions within the catalyst structure that support Cu adherence to the matrix, potentially reducing the tendency for Cu leaching seen at both lower and higher Rb concentrations. This stabilizing interaction could be due to Rb ions at 4 wt% facilitating a favorable electrostatic environment or anchoring effect that retains Cu within the matrix, thereby minimizing its dissolution or detachment during synthesis or reaction. In contrast, lower Rb loadings may not provide sufficient stabilization, while higher Rb loadings might promote competitive ion exchange or destabilize Cu adherence to the support. These observations underscore the importance of optimizing Rb content in the CuZnFe catalyst to balance Cu stability and prevent leaching, ultimately enhancing the catalytic performance in $CO_2$ hydrogenation reactions.

**Table 1** above shows that the specific BET surface area ($S_{BET}$) of CuZnFe increases significantly with the introduction of Rb, with the 2%Rb/CuZnFe sample showing the highest value at 60.0 $m^2$/g. This is almost double that of the parent CuZnFe catalyst (31.7 $m^2$/g), suggesting that Rb addition enhances the surface area. However, further increases in Rb content lead to a decrease in surface area, with 4%Rb/CuZnFe exhibiting the lowest $S_{BET}$ at 23.0 $m^2$/g. This could be due to excessive Rb loading, potentially causing partial blockage of pores or pore collapseWhen the Rb loading was increased to 8%, the $S_{BET}$ rose to 41.2 $m^2$/g. This increase suggests that while excessive Rb can lead to pore blockage at intermediate levels (as observed with the 4%Rb/CuZnFe catalyst), higher Rb concentrations might induce structural reorganization or the formation of new mesopores. This effect could be related to changes in crystalline phases or the redistribution of Rb at higher loadings, which can enhance surface exposure and improve the overall porosity of the catalyst. While the increase in $S_{BET}$ at 2 wt% alkali metal loading appears significant, the consistent observation of this trend across various alkali-modified samples (not limited Rb) suggests a systematic effect. However, we acknowledge that the underlying mechanism may involve subtle changes in crystallization dynamics, metal dispersion, or particle agglomeration during synthesis. Therefore, we refrain from over-interpreting this trend and do not attribute it solely to a 'textural promotion' effect by the alkali metal.

The mesoporous surface area ($S_{meso}$) follows a similar trend, with the 2%Rb/CuZnFe catalyst showing the highest mesoporous surface area (56.2 m$^2$/g). This value sharply drops with higher Rb content, indicating that higher Rb doping might alter the mesoporous structure, reducing its overall effectiveness for certain catalytic processes. The microporous surface area ($S_{micro}$), although relatively small, shows a gradual increase with increasing Rb content, which suggests some degree of pore structure modification. The total pore volume ($V_{total}$) and mesoporous volume ($V_{meso}$) show an interesting pattern. Despite the increase in surface area for the 2%Rb/CuZnFe sample, its total pore volume decreases slightly compared to CuZnFe, from 0.149 to 0.123 cm$^3$/g. This is likely due to changes in pore structure, as indicated by the shift in PD discussed below. The 4%Rb/CuZnFe catalyst shows a slightly reduced pore volume, while 8%Rb/CuZnFe has the lowest total pore volume (0.088 cm$^3$/g), possibly due to further structural collapse or compaction caused by the high Rb loading.

The average PD shows substantial variation with Rb addition. The parent CuZnFe catalyst has an average PD of 15.5 nm, but this drastically decreases to 6.1 nm for the 2%Rb/CuZnFe catalyst and to 5.9 nm for the 8%Rb/CuZnFe sample. This decrease suggests that Rb doping influences the pore size distribution by narrowing the pores, which is consistent with the BJH pore size distribution data **(Fig. 11b).** Interestingly, the 4%Rb/CuZnFe sample shows a larger PD of 16.6 nm, which deviates from the general trend. This could indicate that at moderate Rb doping levels, there is a restructuring or reorganization of the pore system, possibly due to Rb acting as a template for larger mesopores.

The XRD patterns displayed in **Fig. 11(c,d)** exhibit the profiles of the CuZnFe and xRb/CuZnFe catalysts. The primary crystalline constituents for the catalysts prior to the reaction encompass ZnO (COD No. 96-900-4182) with peaks at $2\theta$ angles of 31.8°, 34.5°, 36.3°, 47.6°, 56.7°, 62.9°, 66.4°, and 68.0°; CuO (COD No. 96-901-6327) with peaks at $2\theta$ angles of 35.6°, 38.9°, 48.4°, 58.4°, 61.7°, 66.4°, and 68.0°; $Fe_2O_3$ (COD No. 96-154-6384) with peaks at $2\theta$ angles of 24.2°, 33.3°, 35.6°, 40.9°, 49.6°, 54.1°, 62.5°, and 63.9°; as well as $RbNO_3$ (COD No. 96-210-7327) with peaks at $2\theta$ values of 20.7°, 29.5°, 42.2°, 52.3°, 61.3°, 65.4°, and 69.4°.

The presented data in **Table 1** and **Fig. 11 (c,d)** illustrate the effects of varying Rb loadings on the crystallite size and RC of CuZnFe-based catalysts. The results highlight significant structural changes as Rb content increases, which has implications for catalyst stability and performance. XRD analysis revealed that all catalysts maintained similar crystallite sizes in the range of 18.4-21.3 nm, with minor variations that fall near the instrumental error margin. However, the 4%Rb/CuZnFe catalyst exhibited a crystallite size of 21.3 nm and a RC of 95.9%, nearly identical to the unmodified CuZnFe catalyst (RC = 96.5%), indicating excellent structural preservation. This near-complete retention of crystallinity suggests that 4%Rb loading achieves an optimal balance between introducing Rb and maintaining structural order. Such stability is likely advantageous for catalytic performance, as it preserves active sites and reduces the risk of deactivation due to structural degradation. In contrast, the 8%Rb/CuZnFe catalyst showed a notable decrease in RC to 76.1%, suggesting partial structural disorder at higher Rb loading. This trend highlights that moderate Rb incorporation maintains crystalline integrity, while excessive Rb may disrupt the structure and weaken the catalyst stability and reduce its performance in catalytic applications by diminishing the number of accessible active sites and increasing the likelihood of deactivation.

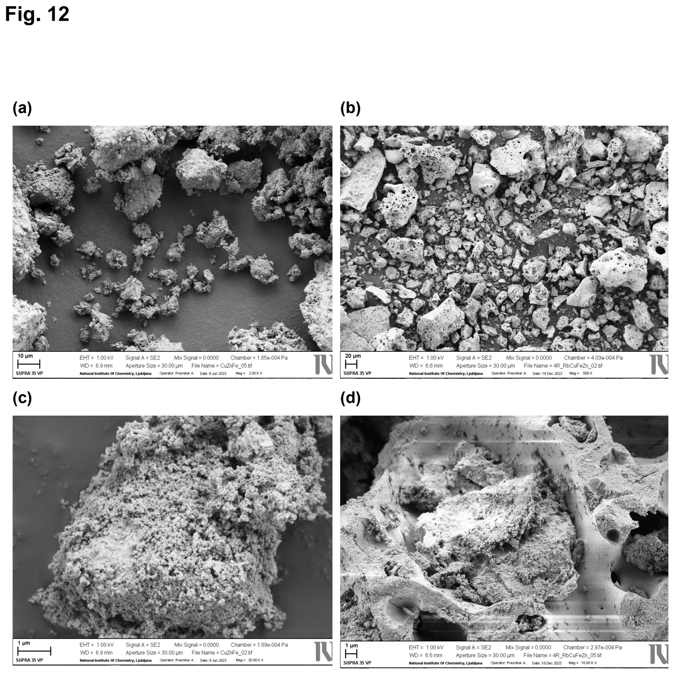

The SEM images provide detailed insights into the morphological differences between the CuZnFe catalyst and the 4%Rb/CuZnFe catalyst (Fig. 12), revealing key structural features that underpin the enhanced catalytic performance of the Rb-modified catalyst. The CuZnFe catalyst **(Fig. 12a, Fig. 12c)** demonstrates a relatively uniform distribution of particles with varying sizes. The particles exhibit aggregation, forming larger clusters with irregular shapes. The surface texture is notably rough, which is generally indicative of relatively high surface area, which is a beneficial characteristic for catalytic activity. However, the extent of aggregation in the CuZnFe sample could limit the accessibility of reactants to active sites, potentially constraining its catalytic efficiency.

In contrast, the 4%Rb/CuZnFe catalyst **(Fig. 12, Fig. 12)** exhibits significant morphological transformations following the incorporation of Rb. The particles display a more open and porous sponge-like structure, with reduced aggregation compared to the CuZnFe catalyst. This increased porosity is particularly advantageous, as it enhances the accessibility of reactants to the active catalytic sites, thereby improving catalytic activity and selectivity. The smoother surface of the 4%Rb/CuZnFe particles compared to the unmodified catalyst may also facilitate more uniform interactions with reactants, which could contribute to the improved catalytic performance observed in this study.

The presence of Rb in the catalyst may facilitate this sintering process, leading to structural modifications that improve the mechanical stability of the catalyst while minimizing issues like leaching of active components. Despite the potential for sintering to reduce surface area, the enhanced porosity and structural integrity observed in the Rb-modified catalyst suggest that the Rb content has been optimized to balance these effects. The open structure ensures adequate access to active sites, while the increased thermal stability minimizes deactivation over time. The observed morphological changes align closely with the enhanced catalytic performance metrics of the 4%Rb/CuZnFe catalyst.

The elemental composition of the CuZnFe and xRb/CuZnFe catalysts was analysed using SEM-EDX, as summarized in **Table 4.**

**Table 4.** SEM-EDX analysis of the CuZnFe and xRb/CuZnFe catalysts.

| CuZnFe | | | |
|---|---|---|---|
| Element | Average (wt%) | Scan 1 (wt%) | Scan 2 (wt%) | Scan 3 (wt%) |
| Cu | 20.1 | 20.5 | 20.0 | 19.7 |
| Fe | 35.6 | 35.8 | 35.7 | 35.4 |
| O | 29.7 | 28.9 | 29.7 | 30.5 |
| Zn | 14.6 | 14.8 | 14.6 | 14.4 |
| **2%Rb/CuZnFe** | | | |
| Rb | 2.3 | 2.2 | 2.1 | 2.4 |
| Cu | 15.3 | 16.0 | 15.4 | 14.7 |
| Fe | 40.6 | 40.2 | 41.3 | 40.4 |
| O | 21.0 | 20.5 | 20.8 | 21.8 |
| Zn | 20.8 | 21.1 | 20.5 | 20.7 |
| **4%Rb/CuZnFe** | | | |
| Rb | 1.9 | 2.5 | 1.5 | 1.6 |
| Cu | 20.5 | 20.4 | 20.7 | 20.4 |
| Fe | 36.9 | 36.4 | 37.2 | 37.0 |
| O | 26.1 | 25.8 | 26.1 | 26.5 |
| Zn | 14.6 | 14.9 | 14.4 | 14.5 |
| **8%Rb/CuZnFe** | | | |
| Rb | 4.4 | 4.4 | 4.5 | 4.3 |
| Cu | 13.4 | 13.2 | 13.6 | 13.4 |
| Fe | 38.8 | 38.1 | 38.8 | 39.4 |
| O | 23.9 | 24.4 | 23.7 | 23.8 |
| Zn | 19.5 | 19.9 | 19.5 | 19.2 |

The pristine CuZnFe catalyst exhibits an average Cu content of 20.1 wt%, Fe at 35.6 wt%, Zn at 14.6 wt%, and O at 29.7 wt%. The relatively high Fe content suggests its dominant presence in the catalyst structure, likely contributing to the catalyst redox properties. The Zn fraction remains stable across multiple scans, indicating a homogeneous distribution of Zn within the catalyst matrix. Upon modification with Rb, notable compositional changes occur. The introduction of 2% Rb leads to a decrease in Cu content to 15.3 wt%, accompanied by an increase in Fe (40.6 wt%) and Zn (20.8 wt%). The incorporation of Rb may alter the surface dispersion of active metal sites, thereby influencing metal-support interactions. The oxygen content remains within the expected range (21.0 wt%), suggesting that oxidation state variations upon Rb addition are minimal. At the highest Rb loading (8%), the Cu content drops significantly to 13.4 wt%, while Fe and Zn increase to 38.8 wt% and 19.5 wt%, respectively. The oxygen content also declines to 23.9 wt%, which may be linked to structural modifications induced by Rb incorporation. The higher Rb content (4.4 wt%) suggests more successful deposition compared to lower loading levels. The increased Fe fraction at higher Rb loadings implies that Rb may influence the catalyst surface chemistry, potentially by altering the oxidation states of Fe and Cu.

Interestingly, increasing the Rb loading to 4 wt% results in a notable increase in Cu content to 20.5 wt%, while Fe and Zn remain comparable at 36.9 wt% and 14.6 wt%, respectively. The oxygen content decreases slightly to 26.1 wt%, which may indicate structural modifications due to the presence of Rb. However, the Rb content (1.9 wt%) is lower than the nominal loading, suggesting limitations in Rb incorporation or possible leaching effects during the preparation process. The increase in Cu content for 4%Rb/CuZnFe, compared to all other studied samples, suggests that 4wt%Rb provides an optimal environment for Cu stabilization. At this concentration, Rb may promote stronger metal-support interactions, reducing Cu mobility and preventing its migration or sintering during catalyst synthesis. In contrast, at lower Rb loadings (2%Rb), this stabilization effect is less pronounced, leading to lower Cu retention. At higher Rb loadings (8%Rb), excessive Rb may disrupt the catalyst structure or induce phase segregation, thereby reducing Cu incorporation. Additionally, Rb is known to alter the electronic properties of metal oxides, influencing the deposition and stabilization of other metals. At an optimal 4%Rb, the modified surface charge may enhance Cu anchoring, preventing Cu leaching or loss during synthesis. However, at higher Rb loadings (8%Rb), excessive Rb could introduce excessive structural changes, potentially reducing Cu incorporation. Thus, these results suggest that the 4%Rb/CuZnFe catalyst exhibits a unique composition where Cu retention is maximized, likely due to an optimal balance between Rb-induced stabilization and structural integrity.

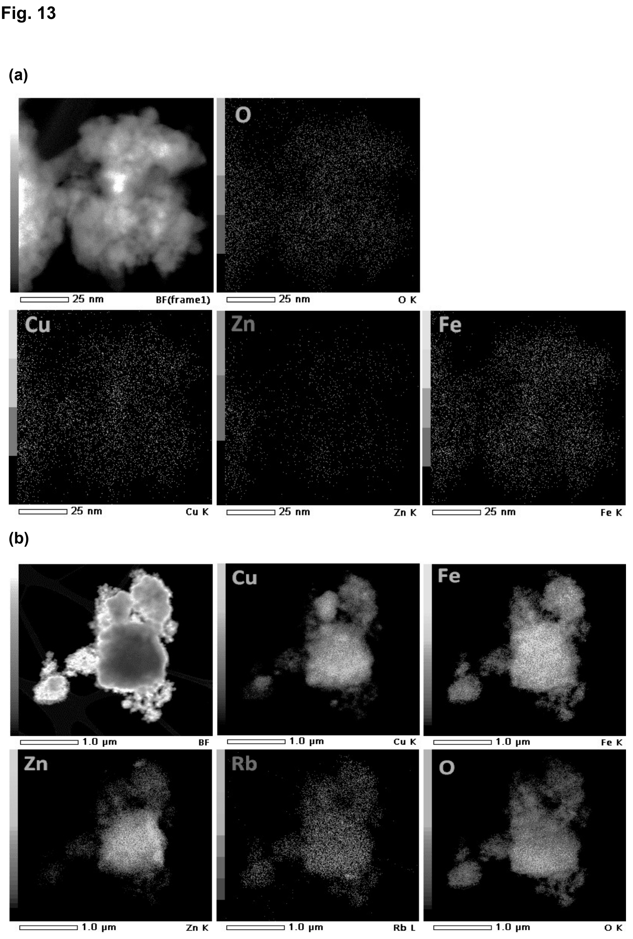

The structural and compositional characteristics of the CuZnFe and 4%Rb/CuZnFe catalysts were investigated using STEM-EDX elemental mapping (Fig. 13) and STEM-BF imaging **(Fig. 19),** revealing significant modifications upon Rb incorporation. The bright-field STEM image of the CuZnFe catalyst **(Fig. 13a)** shows a highly agglomerated morphology with well-dispersed nanoparticles. EDX elemental mapping confirms the homogeneous distribution of Cu, Zn, Fe, and O across the catalyst, suggesting a well-integrated multi-metallic system. The even dispersion of these elements is essential for ensuring uniform active site distribution and optimal catalytic performance. However, the introduction of Rb into the catalyst matrix **(Fig. 13b and 19b)** results in notable morphological and compositional changes. The bright-field STEM image of the 4%Rb/CuZnFe sample reveals larger and more aggregated structures compared to the pristine CuZnFe catalyst, suggesting an influence of Rb on particle growth and sintering behaviour. The EDX elemental mappings confirm the presence of Rb, which appears well-dispersed throughout the sample, indicating successful incorporation into the catalyst structure. The redistribution of elements upon Rb modification may significantly influence catalytic behaviour. The presence of Rb can alter the electronic properties of Cu and Fe species, affecting their reducibility and metal-support interactions.

Further insights into the structural evolution induced by Rb addition are provided by high-resolution STEM-BF images **(Fig. 19).** The CuZnFe catalyst **(Fig. 19a)** exhibits a dispersed nanoparticulate structure with no visible signs of sintering or large crystalline domains. The small particle size and relatively high dispersion suggest a high surface area, which is typically beneficial for catalytic performance due to enhanced accessibility of active sites. However, upon modification with Rb, the 4%Rb/CuZnFe catalyst displays more pronounced morphological changes. A distinctive hexagonal particle is observed **(Fig. 19b),** suggesting the formation of a secondary crystalline phase. This structural transformation may indicate the segregation of certain elements or the emergence of a new phase that alters the catalyst physicochemical properties. The increased particle size and the formation of well-defined crystallites suggest that Rb incorporation may induce restructuring effects, potentially modifying the accessibility and stability of active sites. Thus, the observed changes in particle morphology, elemental distribution, and potential phase segregation suggest that Rb serves not only as an electronic promoter but also as a structural modifier. These effects could impact catalytic activity, selectivity, and stability in $CO_2$ hydrogenation reactions.

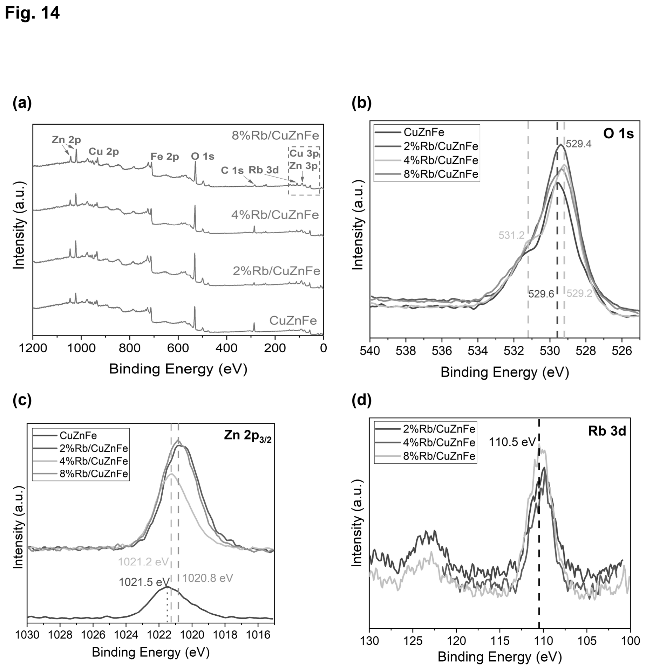

X-ray photoelectron spectroscopy (XPS) measurements were conducted to investigate the elemental composition and electronic states of the CuZnFe and xRb/CuZnFe catalysts, as presented in **Fig. 14.** The survey spectra **(Fig. 14a)** confirm the presence of Cu, Zn, Fe, O, and Rb in the catalysts, with no detectable impurities, indicating the high purity of the synthesized materials. The O 1s spectra **(Fig. 14b)** exhibit a prominent peak centred around 529.6-531.2 eV, which can be attributed to lattice oxygen and surface-adsorbed oxygen species. The primary peak at approximately 529.2-529.6 eV corresponds to metal-oxide bonds (M-O), while the higher binding energy (BE) component at ~531.2 eV is typically associated with hydroxyl species and adsorbed oxygen. Notably, the O 1s peak shifts slightly with increasing Rb content, suggesting modifications in the electronic environment, possibly due to changes in oxygen vacancies or altered metal-oxygen interactions upon Rb incorporation. The most pronounced shift was observed for the 4%Rb/CuZnFe catalyst, with the main O 1s peak appearing at 529.2 eV, along with the most prominent shoulder at 531.2 eV. This catalyst also exhibited the highest activity in $CO_2$ hydrogenation, yielding ethanol and HA, which aligns with the observed electronic modifications. The correlation between electronic structure changes and catalytic performance suggests that Rb plays a crucial role in tuning the active sites and enhancing the reaction pathway for selective alcohol synthesis.

The Zn 2p spectra **(Fig. 14c)** exhibit the Zn $2p_{3/2}$ peak at 1020.8-1021.5 eV, which is characteristic of $Zn^{2+}$ in ZnO. The variation in BE among the samples suggests a possible alteration in the Zn electronic structure upon Rb incorporation. Notably, the Zn 2p peak shifts to lower BE in the Rb-modified catalysts, decreasing from 1021.5 to 1020.8 eV, indicating a change in the local electronic environment of Zn. This shift suggests an increased electron density around Zn, potentially due to charge redistribution or modified metal-support interactions induced by Rb addition. While the shift is observed across all Rb-containing samples, it is less pronounced in the 4%Rb/CuZnFe catalyst, implying this loading, the electronic effects may be counterbalanced by other structural modifications. The Rb 3d region **(Fig. 14d)** reveals a peak centred around 110.5 eV, confirming the successful incorporation of Rb into the catalyst structure. The consistent peak position across the Rb-containing samples suggests that Rb exists in a similar oxidation state in all compositions, likely as $Rb^+$ species interacting with the CuZnFe surface.

The Cu 2p XPS spectra **(Fig. 20)** reveal the Cu $2p_{3/2}$ and Cu $2p_{1/2}$ core-level peaks centred at approximately 933.3 eV and 953.5 eV, respectively. These BE values are characteristic of $Cu^{2+}$ species in CuO, confirming that copper in the catalysts predominantly exists in an oxidized state. The absence of a strong satellite peak at higher BE further supports the dominance of $Cu^{2+}$ over metallic $Cu^0$ species. The CuZnFe sample exhibits significantly lower peak intensity compared to the Rb-modified catalysts, indicating a lower surface concentration of Cu species. Upon Rb addition, the Cu 2p peak intensities increase, suggesting enhanced Cu surface exposure and potential modifications in the electronic structure. This increase in intensity is particularly noticeable for the 2%Rb/CuZnFe and 8%Rb/CuZnFe samples. However, in the 4%Rb/CuZnFe catalyst, the Cu 2p signal remains strong but exhibits a lower intensity compared to the other Rb-containing samples. The BE of Cu $2p_{3/2}$ remains nearly constant across all Rb-modified samples, indicating that Rb incorporation does not significantly alter the oxidation state of Cu but may influence its electronic environment. Notably, a small shift in BE is observed for the 4%Rb/CuZnFe sample, where the Cu $2p_{1/2}$ peak shifts from 953.5 eV to 952.6 eV. This shift, along with variations in peak intensity and shape, suggests electronic interactions between Cu and Rb species, which could influence catalytic performance. The surface composition of CuZnFe and xRb/CuZnFe catalysts, as determined by XPS analysis, is presented in **Tables 5** and **6.**

**Table 5** Surface composition of CuZnFe and xRb/CuZnFe catalysts as determined by XPS analysis.

| Catalyst | C 1s, at.% | O 1s, at.% | Fe 2p, at.% | Cu 2p, at.% | Zn 2p, at. % | Rb 3d, at.% |
|---|---|---|---|---|---|---|
| CuZnFe | 37.4 | 44.5 | 8.2 | 5.0 | 4.9 | 0.0 |
| 2%Rb/CuZnFe | 13.2 | 56.0 | 10.2 | 9.8 | 8.6 | 2.2 |
| 4%Rb/CuZnFe | 28.4 | 51.0 | 8.8 | 4.8 | 5.2 | 1.8 |
| 8%Rb/CuZnFe | 10.2 | 58.2 | 10.2 | 9.6 | 8.5 | 3.3 |

**Table 6** Surface composition of CuZnFe and xRb/CuZnFe catalysts as determined by XPS analysis without C 1s.

| Catalyst | O 1s, at.% | Fe 2p, at.% | Cu 2p, at.% | Zn 2p, at.% | Rb 3d, at.% |
|---|---|---|---|---|---|
| CuZnFe | 71.1 | 13.1 | 8.0 | 7.8 | 0.0 |
| 2%Rb/CuZnFe | 64.5 | 11.8 | 11.3 | 9.9 | 2.5 |
| 4%Rb/CuZnFe | 71.2 | 12.3 | 6.7 | 7.3 | 2.5 |
| 8%Rb/CuZnFe | 64.8 | 11.4 | 10.7 | 9.5 | 3.7 |

Overall, the XPS results provide strong evidence that Rb is effectively incorporated into the CuZnFe catalyst without introducing impurities. The observed shifts in BE, particularly in the O 1s, Zn 2p, and Cu 2p spectra, indicate that Rb influences the electronic properties of the catalyst, which could affect its catalytic performance.

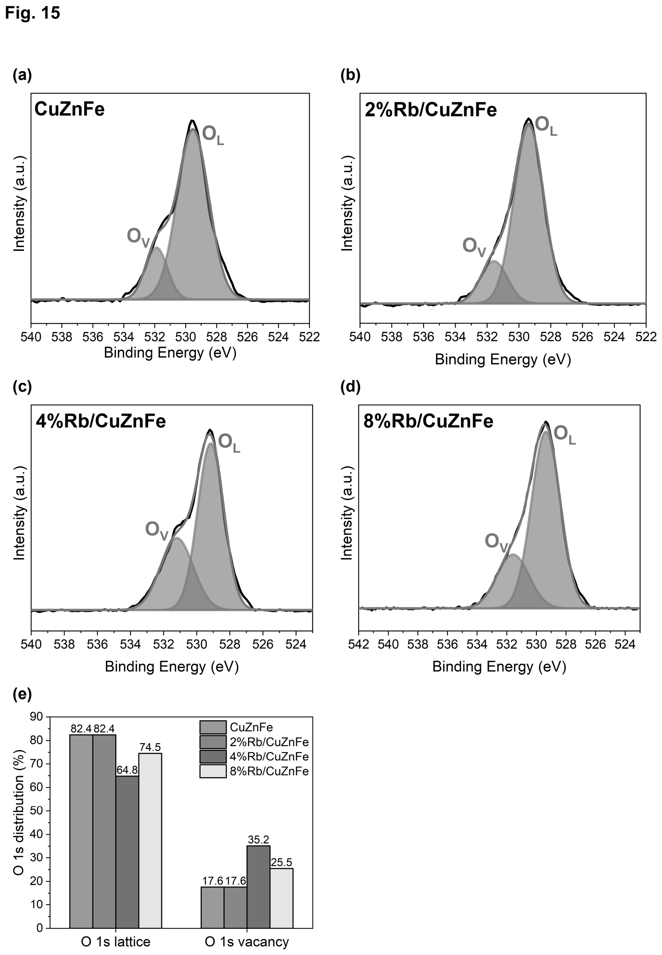

To gain deeper insights into the surface oxygen species and their distribution in CuZnFe and xRb/CuZnFe catalysts, the O 1s XPS spectra were deconvoluted, as shown in **Fig. 15.** The spectra reveal two distinct oxygen species: lattice oxygen ($O_L$) at approximately 529.0-530.0 eV and oxygen vacancies or adsorbed oxygen species ($O_V$) appearing at higher BE (~531.0-531.5 eV). The relative contributions of these components provide crucial information regarding the influence of Rb on the electronic environment and defect structure of the catalysts. The pristine CuZnFe catalyst **(Fig. 15a)** exhibits a dominant $O_L$ peak, indicating a well-structured metal-oxide framework with a low concentration of oxygen vacancies.

With the incorporation of Rb **(Fig. 15b-d),** notable shifts in the $O_L/O_V$ ratio are observed. Specifically, the percentage of $O_V$ increases with Rb doping up to 4%Rb/CuZnFe but decreases slightly for 8%Rb/CuZnFe **(Fig. 15e).** This suggests that Rb addition initially promotes the formation of oxygen vacancies, which are known to enhance catalytic activity by facilitating oxygen mobility and reactant activation. The maximum $O_V$ concentration (35.2%) is observed for the 4%Rb/CuZnFe sample, whereas a decrease to 25.5% for 8%Rb/CuZnFe suggests partial passivation or structural stabilization at higher Rb loading. The trend in $O_V$ formation implies that moderate Rb incorporation induces defect sites, which could enhance redox properties and catalytic performance. However, excessive Rb loading may lead to surface restructuring or occupation of active sites, thereby reducing the concentration of $O_V$. The observed variations in O 1s BE and distribution further confirm the electronic interactions between Rb and the metal-oxide framework.

The adsorption and activation of $CO_2$ are crucial factors influencing the catalytic performance of CuZnFe and Rb-modified CuZnFe catalysts in $CO_2$ hydrogenation reactions. To assess the adsorption properties, $CO_2$-TPD measure-

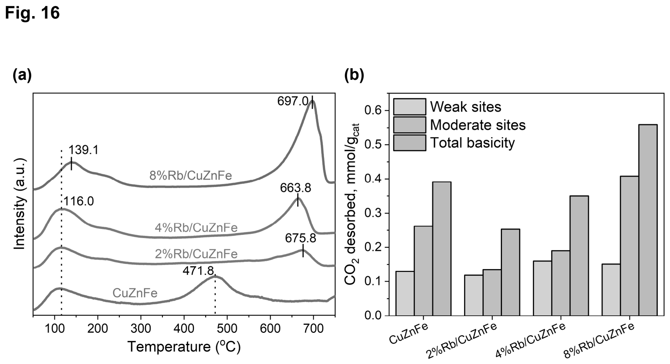

ments were performed, as shown in **Fig. 16.** The desorption profiles **(Fig. 16a)** reveal three distinct temperature regions corresponding to different types of basic sites: the low-temperature region (50-300 °C), attributed to weak basic sites ($CO_2$ adsorption on hydroxyl groups); the medium-temperature region (300-570 °C), associated with moderate basic sites (oxygen vacancies or surface defects), observed only for the unmodified CuZnFe catalyst; and the high-temperature region (above 570 °C), assigned to strong basic sites (coordination-unsaturated $O^{2-}$ species). The $CO_2$-TPD results show significant variations in $CO_2$ adsorption capacity with increasing Rb content. The unmodified CuZnFe catalyst exhibits moderate $CO_2$ desorption, with a prominent peak at 471.8 °C, indicating the presence of moderate basic sites. The incorporation of 2%Rb enhances $CO_2$ adsorption, particularly in the high-temperature range (675.8 °C), suggesting an increase in oxygen vacancies that facilitate $CO_2$ activation. Interestingly, the 4%Rb/CuZnFe catalyst exhibits a shift in the high-temperature desorption peak to a slightly lower temperature (663.8 °C), indicating the presence of relatively weaker basic sites. However, its total $CO_2$ adsorption capacity is slightly higher than that of the 2%Rb/CuZnFe catalyst, suggesting that modifications in the surface composition influence basicity and catalytic performance. The 8%Rb/CuZnFe catalyst exhibits the highest total $CO_2$ desorption, with a dominant high-temperature peak at 697.0 °C, confirming the formation of highly stable strong basic sites. These strong sites, attributed to coordination-unsaturated $O^{2-}$ species, are particularly crucial for $CO_2$ activation and hydrogenation. However, an excessive number of strong basic sites may lead to over-adsorption of $CO_2$, potentially limiting catalytic efficiency due to restricted site availability and increased formation of side products. The quantitative $CO_2$ desorption data **(Fig. 16b)** further confirm these trends. The 2%Rb/CuZnFe catalyst shows a significant increase in total $CO_2$ adsorption compared to unmodified CuZnFe, with a balanced distribution of weak and moderate basic sites. The 4%Rb/CuZnFe catalyst, while exhibiting slightly higher total $CO_2$ adsorption, displays an increased proportion of strong basic sites, which may enhance selective $CO_2$ hydrogenation. In contrast, the 8% Rb/CuZnFe catalyst shows the highest total basicity, primarily due to the abundance of strong basic sites, which may enhance $CO_2$ activation but also pose selectivity challenges. The $CO_2$- TPD results demonstrate that Rb incorporation significantly modifies the basic properties of CuZnFe-based catalysts, with moderate Rb loading (2-4 wt%) providing an optimal balance between weak and strong basic sites. While the 8%Rb/CuZnFe catalyst exhibits the highest basicity, excessive $CO_2$ adsorption may negatively impact selectivity in $CO_2$ hydrogenation reactions. These findings emphasize the importance of optimizing Rb content to achieve the best catalytic performance by carefully balancing $CO_2$ adsorption strength and active site availability.

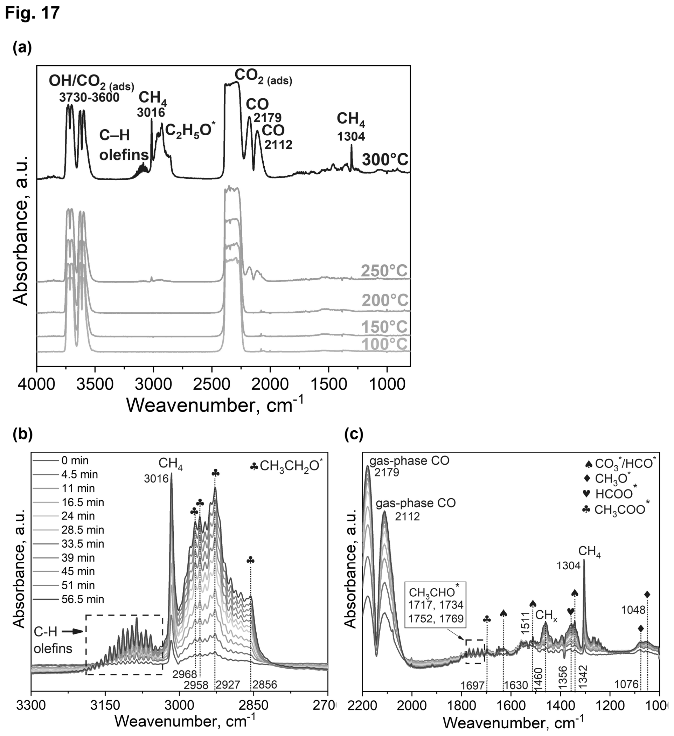

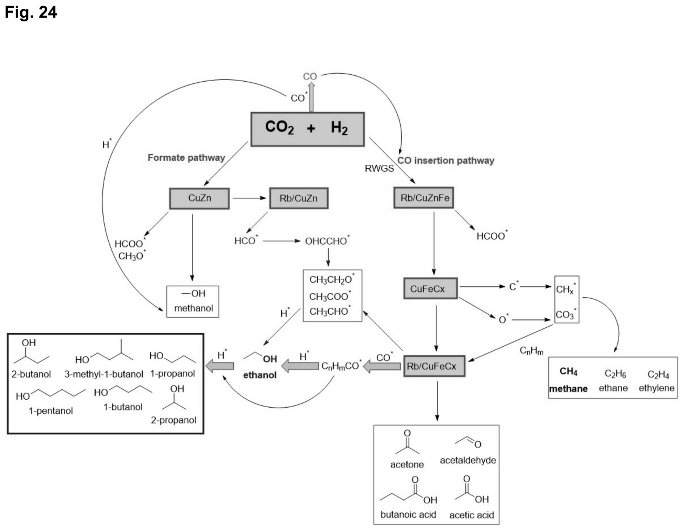

The reaction mechanism of $CO_2$ hydrogenation over the 4%Rb/CuZnFe catalyst, as illustrated in **Fig. 24,** proceeds via two primary pathways: the formate pathway and the CO insertion pathway, both of which are confirmed by *in situ* DRIFTS analysis presented in **Fig. 17.** The prominent IR absorption band in the 2400-2200 $cm^{-1}$ range can be attributed to the presence of gaseous $CO_2$. The reaction begins with $CO_2$ activation on the catalyst surface, forming $C0_3^*$ and $HCO^*$ species, as confirmed by the absorption bands at 1628 and 1511 $cm^{-1}$ in **Fig. 17.** The presence of intense gaseous CO signals at 2112 and 2179 $cm^{-1}$ indicates that the RWGS reaction proceeds rapidly at elevated temperatures (250-300 °C), facilitating the reduction of $CO_2$ to CO. This reaction is essential for further carbon chain growth, as CO acts as a key reactant in both hydrocarbon and oxygenate formation. The role of Rb promotion is particularly significant, as it increases surface alkalinity and enhances the adsorption of $CO_2$, stabilizing crucial intermediates such as $HCO^*$ and $CO_3$.

In the formate pathway, $CO_2$ undergoes sequential hydrogenation to form $HCOO^*$ and $CH_30^*$ species, as evidenced by the characteristic bands at 1356, 1076 and 1048 $cm^{-1}$ in **Fig. 17.** The presence of methoxy species is a critical step toward methanol formation, while the formation of $CH_3CH_20^*$ species at 2968, 2958, 2927, and 2856 $cm^{-1}$ suggests that C-C coupling is facilitated under the reaction conditions. Given that the ethanol formation rate significantly exceeded that of methanol, as confirmed by micro-GC and GC-MS analyses, and considering the potential overlap of $CH_3CH_20^*$ and $CH_2O^*$ bands in the 2940-2980 $cm^{-1}$ region, these peaks were assigned to $CH_3CH_2O^*$, a key intermediate in ethanol synthesis. The progressive increase in their intensity with temperature and reaction time further supports a pathway involving the $OHCCHO^*$ intermediate, which undergoes subsequent hydrogenation to yield ethanol. The Rb-modified CuZn sites enhance the stabilization of these intermediates, promoting ethnol selectivity over methanation.

The CO insertion pathway represents an alternative route for $C_{2+}$ oxygenate formation, in which CO reacts with Fe-based sites, leading to the formation of $FeC_x$ or chemisorbed CO species. Although spent catalyst characterization was not conducted in this study, we acknowledge that $FeC_x$ species may form under reaction conditions and contribute to C-C coupling and CO insertion. This is consistent with previously reported systems. The formation of $CH_3CHO^*$ and $CH_3CH_20^*$ intermediates in our DRIFTS spectra may indirectly support the involvement of such species. The *in situ* DRIFTS spectra in **Fig. 17** reveal the presence of $CH_3CHO^*$ bands at 1717, 1734, 1752, and 1769 $cm^{-1}$, confirming that CO insertion leads to aldehyde formation. The subsequent hydrogenation of acetaldehyde results in ethanol formation, while further condensation or oxidation pathways can generate higher oxygenates such as acetone, butanoic acid, and acetic acid. The presence of additional carbonate species and methoxy/formate species further supports the idea that Rb plays a role in stabilizing these intermediates, preventing over-hydrogenation to methane.

The DRIFTS spectra also indicate competing pathways, including methanation and hydrocarbon (olefins) formation, particularly at higher temperatures. The peaks at 3016 and 1304 $cm^{-1}$ in **Fig. 17,** attributed to $CH_4$, suggest that methane formation becomes more pronounced with increasing temperature or reaction time. This is further supported by the presence of $CH_x$-related bands at 1460 cm$^{-1}$ in **Fig. 17,** indicating that a portion of the $CO_2$-derived CO undergoes complete hydrogenation rather than C-C coupling. Additionally, the detection of ethane and ethylene **(Fig. 9)** indicates that a fraction of CO intermediates undergoes Fischer-Tropsch pathways, resulting in hydrocarbon formation. This is further supported by the presence of IR bands in the 3190-3030 cm$^{-1}$ region, which are characteristic of C-H stretching vibrations in olefins. The reaction mechanism over xRb/CuZnFe is governed by a delicate balance of $CO_2$ activation, RWGS, C-C coupling, and hydrogenation, where moderate Rb loading enhances $CO_2$ adsorption and stabilizes key intermediates, directing selectivity toward ethanol and higher oxygenates instead of methane.

1.5 <u>Conclusions</u>

The 4%Rb/CuZnFe catalyst demonstrates optimal performance in $CO_2$ hydrogenation to ethanol, achieving an impressive STY of 4.4 mmol.g$_{cat}^{-1}$·h$^{-1}$ in the gas phase, with a maximum $C_{2+}$OH selectivity of 85.3%. This high performance is attributed to its favourable combination of crystallinity and pore diameter, which together provide abundant active sites and enhanced diffusion, resulting in significantly higher activity compared to other catalysts. Moreover, optimizing Rb content enhances Cu stability and minimizes leaching, highlighting the importance of precise Rb loading for improved catalytic performance in $CO_2$ hydrogenation reactions. The characterization results revealed the significant influence of the electronic properties of the Rb-modified catalyst on catalytic hydrogenation, which played a crucial role in the selective synthesis of ethanol and HA. The comprehensive physico-chemical characterization reveals structural modifications upon Rb addition, indicating its dual role as both an electronic promoter and a structural modifier by influencing particle morphology, elemental distribution, and potential phase segregation. Ethanol and HA formation follow a multi-step pathway involving $CO_2$ activation, CO formation (RWGS), formate/methoxy intermediates, CO insertion, and ethoxide-mediated chain growth. Moderate Rb loading enhances $CO_2$ adsorption and stabilizes key intermediates, steering selectivity toward ethanol and higher oxygenates instead of methanol. This study offers valuable insights into the rational design and optimization of xRb/CuZnFe catalysts for the thermocatalytic conversion of $CO_2$ into ethanol and HA.

**References**

[1] F. Zeng, C. Mebrahtu, X. Xi, L. Liao, J. Ren, J. Xie, H.J. Heeres, R. Palkovits, Catalysts design for higher alcohols synthesis by CO2 hydrogenation: Trends and future perspectives, Appl. Catal. B Environ. 291 (2021) 120073. https://doi.org/10.1016/j.apcatb.2021.120073.

[2] S. De, A. Dokania, A. Ramirez, J. Gascon, Advances in the Design of Heterogeneous Catalysts and Thermocatalytic Processes for CO2 Utilization, ACS Catal. 10 (2020) 14147-14185. https://doi.org/10.1021/acscatal.0c04273.

[3] I.C. Have, J.J.G. Kromwijk, M. Monai, F. Meirer, B.M. Weckhuysen, E.B. Sterk, Uncovering the reaction mechanism behind CoO as active phase for CO2 hydrogenation, Nat. Commun. 13 (2022). https://doi.org/10.1038/s41467-022-27981-x.

[4] A. Kostyniuk, D. Bajec, B. Likozar, One-step synthesis of ethanol from glycerol in a gas phase packed bed reactor over hierarchical alkali-treated zeolite catalyst materials, Green Chem. 22 (2020) 753-765. https://doi.org/10.1039/c9gc03262b.

[5] Y. Wang, K. Wang, B. Zhang, X. Peng, X. Gao, G. Yang, H. Hu, M. Wu, N. Tsubaki, Direct Conversion of CO2 to Ethanol Boosted by Intimacy-Sensitive Multifunctional Catalysts, ACS Catal. 11 (2021) 11742-11753. https://doi.org/10.1021/acscatal.1c01504.

[6] B. An, Z. Li, Y. Song, J. Zhang, L. Zeng, C. Wang, W. Lin, Cooperative copper centres in a metal-organic framework for selective conversion of CO2 to ethanol, Nat. Catal. 2 (2019) 709-717. https://doi.org/10.1038/s41929-019-0308-5.

[7] D. Xu, M. Ding, X. Hong, G. Liu, S.C.E. Tsang, Selective C2+ Alcohol Synthesis from Direct CO2 Hydrogenation over a Cs-Promoted Cu-Fe-Zn Catalyst, ACS Catal. 10 (2020) 5250-5260. https://doi.org/10.1021/acscatal.0c01184.

[8] C. Yang, B. Wang, Y. Wen, M. Fan, Y. Jia, S. Zhou, W. Huang, Composition control of CuFeZn catalyst derived by PDA and its effect on synthesis of C2+ alcohols from CO2, Fuel. 327 (2022) 125055. https://doi.org/10.1016/j.fuel.2022.125055.

[9] G.T. Jaya, R. Insyani, J. Park, A.F. Barus, M.G. Sibi, V. Ranaware, D. Verma, J. Kim, One-pot conversion of lignocellulosic biomass to ketones and aromatics over a multifunctional Cu-Ru/ZSM-5 catalyst, Appl. Catal. B Environ. 312 (2022) 121368. https://doi.org/10.1016/j.apcatb.2022.121368.

[10] A. Kostyniuk, D. Bajec, P. Djinovic, B. Likozar, Allyl alcohol production by gas phase conversion reactions of glycerol over bifunctional hierarchical zeolite-supported bi- and trimetallic catalysts, Chem. Eng. J. 397 (2020) 125430. https://doi.org/10.1016/j.cej.2020.125430.

[11] P. Tian, G. Zhan, J. Tian, K.B. Tan, M. Guo, Y. Han, T. Fu, J. Huang, Q. Li, Direct CO2 hydrogenation to light olefins over ZnZrOx mixed with hierarchically hollow SAPO-34 with rice husk as green silicon source and template, Appl. Catal. B Environ. 315 (2022) 121572. https://doi.org/10.1016/j.apcatb.2022.121572.

[12] Y. Lou, F. Jiang, W. Zhu, L. Wang, T. Yao, S. Wang, B. Yang, B. Yang, Y. Zhu, X. Liu, CeO2 supported Pd dimers boosting CO2 hydrogenation to ethanol, Appl. Catal. B Environ. 291 (2021) 120122. https://doi.org/10.1016/j.apcatb.2021.120122.

[13] L. Ding, T. Shi, J. Gu, Y. Cui, Z. Zhang, C. Yang, T. Chen, M. Lin, P. Wang, N. Xue, L. Peng, X. Guo, Y. Zhu, Z. Chen, W. Ding, CO2 Hydrogenation to Ethanol over Cu@Na-Beta, Chem. 6 (2020) 2673-2689. https://doi.org/10.1016/j.chempr.2020.07.001.

[14] D. Xu, H. Yang, X. Hong, G. Liu, S.C. Edman Tsang, Tandem Catalysis of Direct CO2 Hydrogenation to Higher Alcohols, ACS Catal. 11 (2021) 8978-8984. https://doi.org/10.1021/acscatal.1c01610.

[15] D. Xu, M. Ding, X. Hong, G. Liu, Mechanistic aspects of the role of K promotion on Cu-Fe-based catalysts for higher alcohol synthesis from CO2 hydrogenation, ACS Catal. 10 (2020) 14516-14526. https://doi.org/10.1021/acscatal.0c03575.

[16] G. Zhang, G. Fan, L. Zheng, F. Li, Ga-Promoted CuCo-Based Catalysts for Efficient CO2Hydrogenation to Ethanol: The Key Synergistic Role of Cu-CoGaOxInterfacial Sites, ACS Appl. Mater. Interfaces. 14 (2022) 35569-35580. https://doi.org/10.1021/acsami.2c07252.

[17] H. Guo, S. Li, F. Peng, H. Zhang, L. Xiong, C. Huang, C. Wang, X. Chen, Roles Investigation of Promoters in K/Cu-Zn Catalyst and Higher Alcohols Synthesis from CO2 Hydrogenation over a Novel Two-Stage Bed Catalyst Combination System, Catal. Letters. 145 (2015) 620-630. https://doi.org/10.1007/s10562-014-1446-7.

[18] S. Santanta, M.T.M. Koper, H.M. Alisson, L.H. Vieira, E.M. Assaf, M. Assaf, J.F. Gomes, Ethanol formation from CO2 hydrogenation at atmospheric pressure using Cu catalysts: Water as a key component, Appl. Catal. B, Environ. 324 (2023) 122221. https://doi.org/10.1016/j.apcatb.2022.122221.

[19] M. Takagawa, A. Okamoto, H. Fujimura, Y. Izawa, H. Arakawa, Ethanol synthesis from carbon dioxide and hydrogen, Stud. Surf. Sci. Catal. 114 (1998) 525-528. https://doi.org/10.1016/s0167-2991(98)80812-4.

[20] Z. Si, L. Wang, Y. Han, J. Yu, Q. Ge, C. Zeng, J. Sun, Synthesis of Alkene and Ethanol in CO2 Hydrogenation on a Highly Active Sputtering CuNaFe Catalyst, ACS Sustain. Chem. Eng. 10 (2022) 14972-14979. https://doi.org/10.1021/acssuschemeng.2c05450.

[21] Y. Wang, X. Zhang, X. Hong, G. Liu, Sulfate-Promoted Higher Alcohol Synthesis from CO2 Hydrogenation, ACS Sustain. Chem. Eng. 10 (2022) 8980-8987. https://doi.org/10.1021/acssuschemeng.2c02743.

## Claims

1. A hydrogenation catalyst, comprising or essentially consisting of Cu-Zn-Fe mixed oxide particles loaded with Rb in an amount of 2-8 wt.%, based on the total weight of the loaded Cu-Zn-Fe mixed oxide particles.

2. The catalyst of claim 1, wherein Rb is loaded in an amount of 3-6 mol %, preferably 3.5-5 mol %, in particular about 4 mol %, based on the total weight of the loaded Cu-Zn-Fe mixed oxide particles.

3. The catalyst of claim 1 or 2, comprising

   Cu in an amount of 10-25 wt.%, preferably 18-22 wt.%, more preferably about 20 wt.%, Zn in an amount of 10-25 wt.%, preferably 12-16 wt.%, more preferably about 15 wt.%, and
   Fe in an amount of 33-45 wt.%, preferably 35-38 wt.%, more preferably about 37 wt.%, based on the total weight of the loaded Cu-Zn-Fe mixed oxide particles,
   in particular wherein Cu, Zn and Fe are present in a molar ratio of 1:1-2:2-3, preferably about 1:1:2.

4. The catalyst of any one of the preceding claims, wherein the catalyst is a powder comprising particles with an average particle size in a range of 10-50 nm, preferably 15-30 nm.

5. The catalyst of any one of the preceding claims, wherein the Cu-Zn-Fe mixed oxide particles loaded with Rb are porous particles comprising mesopores and micropores.

6. The catalyst of claim 5, wherein the average pore diameter is in a range of 5-20 nm, preferably 6-18 nm.

7. A method for preparing the hydrogenation catalyst of any one of claims 1-6, comprising:

   (i) providing Cu-Zn-Fe mixed oxide particles;

(ii) impregnating the Cu-Zn-Fe mixed oxide particles with a solution of a Rb salt, preferably an aqueous solution of $RbNO_3$;

(iii) drying the impregnated particles, preferably by heating to a temperature in a range of 50-150°C, more preferably 60-130°C, in particular about 110°C;

(iv) calcination of the dried particles, preferably at a temperature in a range of 300-600°C, more preferably 350-500°C, in particular about 400°C.

8. The method of claim 7, wherein step (ii) further comprises adding one or more additional alkali elements, such as Li, Na, K or combinations thereof, preferably as an aqueous solution comprising a Li, Na, or K salt or a combination thereof.

9. The method of claim 7 or 8, wherein providing Cu-Zn-Fe mixed oxide particles comprises:

(i.1) coprecipitating Cu, Fe and Zn salts from a mixed solution comprising salts of Cu, Fe and Zn, preferably nitrates, in particular in a molar ratio Cu:Zn:Fe of 1:1-2:2-3, preferably about 1:1:2;

(i.2) drying the precipitate, preferably by heating to a temperature in a range of 50-150°C, more preferably 60-130°C, in particular about 110°C and

(i.3) calcination of the dried precipitate, preferably at a temperature in a range of 300-600°C, more preferably 350-500°C, in particular about 400°C.

10. A method for preparing the hydrogenation catalyst of any one of claims 1-6, comprising:

(i) providing a mixed solution comprising salts of Cu, Fe, Zn, and Rb, preferably nitrates, in particular in a molar ratio Cu:Zn:Fe of 1:1-2:2-3, preferably about 1:1:2;

(ii) inducing coprecipitation of Rb-doped CuZnFe precipitate;

(iii) drying the precipitate, preferably by heating to a temperature in a range of 50-150°C, more preferably 60-130°C, in particular about 110°C; and

(iv) calcination of the dried precipitate, preferably at a temperature in a range of 300-600°C, more preferably 350-500°C, in particular about 400°C.

11. The method of claim 10, wherein the mixed solution in step (i) further comprises salts, in particular nitrates, of one or more additional alkali elements, in particular salts of Li, Na, or K, or combinations thereof.

12. A method for preparing ethanol by hydrogenation of $CO_2$, comprising

(i) reacting $H_2$ and $CO_2$ in a reactor the presence of a catalyst according to any one of claims 1-6; and

(ii) isolating ethanol from a reaction product obtained in (i).

13. The method of claim 12, wherein the reaction is carried out in a packed bed continuous flow reactor comprising the catalyst of any one of claims 1-6, and wherein $H_2$ and $CO_2$ are supplied to the reactor with a gas feed rate GHSV in a range of 9000-11000 $h^{-1}$, preferably 9500-10500 $h^{-1}$, more preferably about 10000 $h^{-1}$, and/or a reactor feed rate of 0.01-0.5 g/min, preferably 0.025-0.035 g/min, more preferably about 0.03 g/min or 20-100 ml/min, preferably 50-70 ml/min, more preferably about 60 ml/min.

14. The method of claim 12 or 13, wherein the reaction is carried out at a temperature in a range of 200-400°C, wherein the temperature is preferably increased during the reaction time,

in particular with a first period at a temperature in a range of 180-220°C, preferably for 6-10 h,

increasing the temperature to a higher temperature in a range of 220-270°C and holding the temperature for a second period, preferably for 1-5 h,

increasing the temperature to a higher temperature in a range of 270-350°C and holding the temperature for a third period, preferably for 3-8 h.

15. The method of any one of claims 12-14, wherein the reaction is carried out at a pressure in a range of 15-25 bar, preferably 18-22 bar, more preferably about 20 bar.

**Fig. 1**

**a)**

**b)**

**Fig. 2**

**(a)**

**(b)**

**Fig. 3**

(a)

(b)

(c)

(d)

**Fig. 4**

(a)

(b)

**Fig. 5**

**(a)**

**(b)**

**Fig. 6**

**(a)**

**(b)**

**(c)**

**Fig. 7**

**(a)**

**(b)**

**(c)**

**(d)**

Fig. 8

(a)

(b)

(c)

**Fig. 9**

**(a)**

**(b)**

**Fig. 10**

**(a)**

**(b)**

**(c)**

**(d)**

**Fig. 11**

**(a)**

**(b)**

**(c)**

**(d)**

**Fig. 12**

**(a)**

**(b)**

**(c)**

**(d)**

**Fig. 13**

**(a)**

**(b)**

**Fig. 14**

**Fig. 15**

**Fig. 16**

**(a)**

**(b)**

**Fig. 17**

**(a)**

**(b)**

**(c)**

**Fig. 18**

Filled reactor tube

**Fig. 19**

(a)

(b)

50 nm

50 nm

**Fig. 20**

**Fig. 21**

**Fig. 22**

**Fig. 23**

**Fig. 24**

Europäisches
Patentamt

European
Patent Office

Office européen
des brevets

# EUROPEAN SEARCH REPORT

**Application Number**

EP 25 20 9059

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | XU DI ET AL: "Selective C 2+ Alcohol Synthesis from Direct CO2 Hydrogenation over a Cs-Promoted Cu-Fe-Zn Catalyst", ACS CATALYSIS, vol. 10, no. 9, 9 April 2020 (2020-04-09), pages 5250-5260, XP093262378, US ISSN: 2155-5435, DOI: 10.1021/acscatal.0c01184 * abstract; page 5250 * * page 5251, right-hand column, last paragraph - page 5252, left-hand column, paragraph 1; table 1 * * page 5252, right-hand column, paragraph 2 - page 5253, right-hand column, paragraph 2; figure 2 * * figure 1 * -& XU DI ET AL: "Selective C2+ Alcohol Synthesis from Direct CO2 Hydrogenation over a Cs-Promoted Cu-Fe-Zn Catalyst - Supporting Information", ACS CATALYSIS, vol. 10, no. 9, 9 April 2020 (2020-04-09), XP093262773, DOI: doi:10.1021/acscatal.0c01184 Retrieved from the Internet: URL:https://pubs.acs.org/doi/10.1021/acscatal.0c01184#_i14> * page 2, paragraph 1 * * page 5, paragraph 2 * * figures S5,S13,S16 * ----- -/-- | 1-15 | INV. B01J23/80 B01J37/02 B01J37/03 C07C29/156 C07C31/08 B01J29/46 B01J35/64 |

| | TECHNICAL FIELDS SEARCHED (IPC) |
|---|---|
| | B01J C07C |

The present search report has been drawn up for all claims

| Place of search | Date of completion of the search | Examiner |
|---|---|---|
| Munich | 6 February 2026 | Beckmann, Oliver |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# EUROPEAN SEARCH REPORT

**Application Number**

EP 25 20 9059

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | PAMEI MALA ET AL: "Challenges and advances in understanding the roadmap for direct hydrogenation of carbon dioxide to ethanol", CATALYSIS REVIEWS: SCIENCE AND ENGINEERING, 17 September 2024 (2024-09-17), pages 1-47, XP093262458, US ISSN: 0161-4940, DOI: 10.1080/01614940.2024.2400974 * abstract; page 1 * * figure 21a; table 1 * ----- | 1-15 | |

TECHNICAL FIELDS
SEARCHED (IPC)

The present search report has been drawn up for all claims

| Place of search | Date of completion of the search | Examiner |
|---|---|---|
| Munich | 6 February 2026 | Beckmann, Oliver |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

page 2 of 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **F. ZENG** ; **C. MEBRAHTU** ; **X. XI** ; **L. LIAO** ; **J. REN** ; **J. XIE** ; **H.J. HEERES** ; **R. PALKOVITS**. Catalysts design for higher alcohols synthesis by CO2 hydrogenation: Trends and future perspectives. *Appl. Catal. B Environ*, 2021, vol. 291, 120073, https://doi.org/10.1016/j.apcatb.2021.120073

- **S. DE** ; **A. DOKANIA** ; **A. RAMIREZ** ; **J. GASCON**. Advances in the Design of Heterogeneous Catalysts and Thermocatalytic Processes for CO2 Utilization. *ACS Catal*, 2020, vol. 10, 14147-14185, https://doi.org/10.1021/acscatal.0c04273

- **I.C. HAVE** ; **J.J.G. KROMWIJK** ; **M. MONAI** ; **F. MEIRER** ; **B.M. WECKHUYSEN** ; **E.B. STERK**. Uncovering the reaction mechanism behind CoO as active phase for CO2 hydrogenation. *Nat. Commun.*, 2022, 13, https://doi.org/10.1038/s41467-022-27981-x

- **A. KOSTYNIUK** ; **D. BAJEC** ; **B. LIKOZAR**. One-step synthesis of ethanol from glycerol in a gas phase packed bed reactor over hierarchical alkali-treated zeolite catalyst materials. *Green Chem.*, 2020, vol. 22, 753-765, https://doi.org/10.1039/c9gc03262b

- **Y. WANG** ; **K. WANG** ; **B. ZHANG** ; **X. PENG** ; **X. GAO** ; **G. YANG** ; **H. HU** ; **M. WU** ; **N. TSUBAKI**. Direct Conversion of CO2 to Ethanol Boosted by Intimacy-Sensitive Multifunctional Catalysts. *ACS Catal*, 2021, vol. 11, 11742-11753, https://doi.org/10.1021/acscatal.1c01504

- **B. AN** ; **Z. LI** ; **Y. SONG** ; **J. ZHANG** ; **L. ZENG** ; **C. WANG** ; **W. LIN**. Cooperative copper centres in a metal-organic framework for selective conversion of CO2 to ethanol. *Nat. Catal.*, 2019, vol. 2, 709-717, https://doi.org/10.1038/s41929-019-0308-5

- **D. XU** ; **M. DING** ; **X. HONG** ; **G. LIU** ; **S.C.E. TSANG**. Selective C2+ Alcohol Synthesis from Direct CO2 Hydrogenation over a Cs-Promoted Cu-Fe-Zn Catalyst. *ACS Catal*, 2020, vol. 10, 5250-5260, https://doi.org/10.1021/acscatal.0c01184

- **C. YANG** ; **B. WANG** ; **Y. WEN** ; **M. FAN** ; **Y. JIA** ; **S. ZHOU** ; **W. HUANG**. *Composition control of CuFeZn catalyst derived by PDA and its effect on synthesis of C2+ alcohols from CO2, Fuel*, 2022, vol. 327, 125055, https://doi.org/10.1016/j.fuel.2022.125055

- **G.T. JAYA** ; **R. INSYANI** ; **J. PARK** ; **A.F. BARUS** ; **M.G. SIBI** ; **V. RANAWARE** ; **D. VERMA** ; **J. KIM**. One-pot conversion of lignocellulosic biomass to ketones and aromatics over a multifunctional Cu-Ru/ZSM-5 catalyst. *Appl. Catal. B Environ*, 2022, vol. 312, 121368, https://doi.org/10.1016/j.apcatb.2022.121368

- **A. KOSTYNIUK** ; **D. BAJEC** ; **P. DJINOVIC** ; **B. LIKOZAR**. Allyl alcohol production by gas phase conversion reactions of glycerol over bifunctional hierarchical zeolite-supported bi- and trimetallic catalysts. *Chem. Eng. J.*, 2020, vol. 397, 125430, https://doi.org/10.1016/j.cej.2020.125430

- **P. TIAN** ; **G. ZHAN** ; **J. TIAN** ; **K.B. TAN** ; **M. GUO** ; **Y. HAN** ; **T. FU** ; **J. HUANG** ; **Q. LI**. Direct CO2 hydrogenation to light olefins over ZnZrOx mixed with hierarchically hollow SAPO-34 with rice husk as green silicon source and template. *Appl. Catal. B Environ.*, 2022, vol. 315, 121572, https://doi.org/10.1016/j.apcatb.2022.121572

- **Y. LOU** ; **F. JIANG** ; **W. ZHU** ; **L. WANG** ; **T. YAO** ; **S. WANG** ; **B. YANG** ; **B. YANG** ; **Y. ZHU** ; **X. LIU**. CeO2 supported Pd dimers boosting CO2 hydrogenation to ethanol. *Appl. Catal. B Environ.*, 2021, vol. 291, 120122, https://doi.org/10.1016/j.apcatb.2021.120122

- **L. DING** ; **T. SHI** ; **J. GU** ; **Y. CUI** ; **Z. ZHANG** ; **C. YANG** ; **T. CHEN** ; **M. LIN** ; **P. WANG** ; **N. XUE**. CO2 Hydrogenation to Ethanol over Cu@Na-Beta. *Chem*, 2020, vol. 6, 2673-2689, https://doi.org/10.1016/j.chempr.2020.07.001

- **D. XU** ; **H. YANG** ; **X. HONG** ; **G. LIU** ; **S.C. EDMAN TSANG**. Tandem Catalysis of Direct CO2 Hydrogenation to Higher Alcohols. *ACS Catal*, 2021, vol. 11, 8978-8984, https://doi.org/10.1021/acscatal.1c01610

- **D. XU** ; **M. DING** ; **X. HONG** ; **G. LIU**. Mechanistic aspects of the role of K promotion on Cu-Fe-based catalysts for higher alcohol synthesis from CO2 hydrogenation. *ACS Catal.*, 2020, vol. 10, 14516-14526, https://doi.org/10.1021/acscatal.0c03575

- **G. ZHANG** ; **G. FAN** ; **L. ZHENG** ; **F. LI**. Ga-Promoted CuCo-Based Catalysts for Efficient CO2 Hydrogenation to Ethanol: The Key Synergistic Role of Cu-CoGaOxInterfacial Sites. *ACS Appl. Mater. Interfaces.*, 2022, vol. 14, 35569-35580, https://doi.org/10.1021/acsami.2c07252

- **H. GUO** ; **S. LI, F. PENG** ; **H. ZHANG** ; **L. XIONG** ; **C. HUANG** ; **C. WANG** ; **X. CHEN**. Roles Investigation of Promoters in K/Cu-Zn Catalyst and Higher Alcohols Synthesis from CO2 Hydrogenation over a Novel Two-Stage Bed Catalyst Combination System. *Catal. Letters.*, 2015, vol. 145, 620-630, https://doi.org/10.1007/s10562-014-1446-7

- **S. SANTANTA** ; **M.T.M. KOPER** ; **H.M. ALISSON** ; **L.H. VIEIRA** ; **E.M. ASSAF** ; **M. ASSAF** ; **J.F. GOMES**. Ethanol formation from CO2 hydrogenation at atmospheric pressure using Cu catalysts: Water as a key component. *Appl. Catal. B, Environ.*, 2023, vol. 324, 122221, https://doi.org/10.1016/j.apcatb.2022.122221

- **M. TAKAGAWA** ; **A. OKAMOTO** ; **H. FUJIMURA** ; **Y. IZAWA** ; **H. ARAKAWA**. Ethanol synthesis from carbon dioxide and hydrogen. *Stud. Surf. Sci. Catal.*, 1998, vol. 114, 525-528, https://doi.org/10.1016/s0167-2991(98)80812-4

- **Z. SI** ; **L. WANG** ; **Y. HAN** ; **J. YU** ; **Q. GE** ; **C. ZENG** ; **J. SUN**. Synthesis of Alkene and Ethanol in CO2 Hydrogenation on a Highly Active Sputtering CuNaFe Catalyst. *ACS Sustain. Chem. Eng.*, 2022, vol. 10, 14972-14979, https://doi.org/10.1021/acssuschemeng.2c05450

- **Y. WANG** ; **X. ZHANG** ; **X. HONG** ; **G. LIU**. Sulfate-Promoted Higher Alcohol Synthesis from CO2 Hydrogenation. *ACS Sustain. Chem. Eng.*, 2022, vol. 10, 8980-8987, https://doi.org/10.1021/acssuschemeng.2c02743